# FASCICULE DE BREVET EUROPEEN

(11) **EP 3 649 651 B1**
(45) Date de publication et mention de la délivrance du brevet: **23.09.2026**
(21) Numéro de dépôt: 18735331.3
(22) Date de dépôt: 06.07.2018
(51) Int. Cl.: G16H 50/20

(54) **MÉTHODES D'ANALYSE DE LA DÉRIVE MÉTABOLIQUE CHEZ UN SUJET**
VERFAHREN ZUR ANALYSE VON STOFFWECHSELDRIFT BEI EINER PERSON
METHODS FOR ANALYZING METABOLIC DRIFT IN A SUBJECT

(30) Priorité: 07.07.2017 FR 1756464
(43) Date de publication de la demande: 13.05.2020
(73) Titulaire: Boutin, Ronan, 44400 Reze (FR); BIO LOGBOOK, 44200 Nantes (FR)
(72) Inventeur: BOUTIN, Ronan, 44400 Reze (FR)
(74) Mandataire: Lavoix
(86) Numéro de dépôt international: PCT/EP2018/068430
(87) Numéro de publication internationale: WO 2019/008166

(56) Documents cités:
- EP-A1- 2 645 105
- JP-A- H0 211 129
- US-A1- 2010 203 569
- US-A1- 2016 290 989
- US-B2- 7 912 674
- US-B2- 9 639 667
- PAUL S. HORN ET AL: "Reference intervals: an update", CLINICA CHIMICA ACTA, vol. 334, no. 1-2, 1 August 2003 (2003-08-01), AMSTERDAM, NL, pages 5 - 23, XP055457432, ISSN: 0009-8981, DOI: 10.1016/S0009-8981(03)00133-5
- KIYOSHI ICHIHARA: "Statistical considerations for harmonization of the global multicenter study on reference values", CLINICA CHIMICA ACTA, vol. 432, 1 May 2014 (2014-05-01), AMSTERDAM, NL, pages 108 - 118, XP055507558, ISSN: 0009-8981, DOI: 10.1016/j.cca.2014.01.025
- F. CERIOTTI ET AL: "Reference intervals: the way forward", ANNALS OF CLINICAL BIOCHEMISTRY., vol. 46, no. 1, 1 January 2009 (2009-01-01), GB, pages 8 - 17, XP055457630, ISSN: 0004-5632, DOI: 10.1258/acb.2008.008170
- JIN HWA KIM ET AL: "The Mean Platelet Volume Is Positively Correlated with Serum Thyrotropin Concentrations in a Population of Healthy Subjects and Subjects with Unsuspected Subclinical Hypothyroidism", THYROID., vol. 23, no. 1, 1 January 2013 (2013-01-01), US, pages 31 - 37, XP055457678, ISSN: 1050-7256, DOI: 10.1089/thy.2012.0033
- PAUL S. HORN ET AL: "Reference intervals: an update", CLINICA CHIMICA ACTA, vol. 334, no. 1-2, 1 August 2003 (2003-08-01), AMSTERDAM, NL, pages 5 - 23, XP055457320, ISSN: 0009-8981, DOI: 10.1016/S0009-8981(03)00133-5
- H. C. FORD ET AL: "Haemostasis in hypothyroidism.", POSTGRADUATE MEDICAL JOURNAL., vol. 66, no. 774, 1 April 1990 (1990-04-01), GB, pages 280 - 284, XP055457446, ISSN: 0032-5473, DOI: 10.1136/pgmj.66.774.280
- ALEV AKYOL ERIKCI ET AL: "The effect of subclinical hypothyroidism on platelet parameters", HEMATOLOGY, vol. 14, no. 2, 18 April 2009 (2009-04-18), LX, pages 115 - 117, XP055457706, ISSN: 1024-5332, DOI: 10.1179/102453309X385124

## Description

### Domaine technique

La présente invention concerne des méthodes d'analyse de paramètre(s) biologique(s) d'un sujet.

### Etat de la technique

L'analyse d'un ou plusieurs paramètres biologiques chez un sujet est un outil incontournable d'aide au diagnostic d'une pathologie. En effet, il existe des liens plus ou moins directs entre certains paramètres biologiques et des pathologies données, par exemple entre la glycémie et le diabète, le taux de fer et l'anémie, la tension et l'hypertension, la présence d'un pathogène et une infection liée à ce pathogène, vitesse de sédimentation et une infection ou inflammation, *etc..* La mesure de certains paramètres biologiques permet ainsi d'orienter ou confirmer un diagnostic. Elle permet également de suivre la réponse d'un patient à un traitement, en suivant l'évolution d'un paramètre lié à la maladie concernée. À cet égard, le document EP2645105A1 décrit l'utilisation de biomarqueurs spécifiques, tels que des profils lipidiques, pour prédire le risque d'inflammation de bas grade liée à l'âge en comparant les valeurs mesurées à des niveaux de référence.

L'analyse d'un paramètre biologique consiste généralement à mesurer la valeur de ce paramètre biologique, puis à déterminer si la valeur obtenue est située dans une plage de valeurs de référence définie par une valeur seuil minimale de référence et une valeur seuil maximale de référence. Ainsi, les résultats d'une analyse biologique sont généralement classés en trois catégories : (i) hors valeur seuil de référence minimum, (ii) entre les valeurs seuils de référence minimum et maximum ou (iii) hors valeur seuil de référence maximum. Une valeur se situant à l'intérieur d'une plage de valeurs de référence sera considérée comme « normale ». La « normalité » d'une valeur qui se situe en dehors d'une plage de valeurs de référence est laissée à l'appréciation du praticien, en particulier en fonction de l'écart de cette valeur aux valeurs seuil de référence.

Les valeurs de référence peuvent varier en fonction de l'origine géographique, du sexe et/ou de l'âge d'un sujet et, éventuellement, en fonction de la méthode de dosage utilisée. En général, les valeurs de référence correspondent aux résultats obtenus dans une population de référence dont les individus sont exempts de pathologie et/ou de traitement susceptibles de modifier les valeurs mesurées. Dans la pratique, les valeurs seuils de référence définies par le corps médical ne sont pas nécessairement indexées sur des analyses statistiques de la population.

Les valeurs de référence font l'objet de recommandations internationales, en particulier de l'IFCC-LM (*International Federation of Clinical Chemistry and Laboratory Medicine*) et CLSI (*Clinical and Laboratory Standards Institute*)*.* Dans ce contexte, le document US9639667B2 propose un moteur de test d'intervalles de référence conçu pour établir des plages de normalité à partir de bases de données cliniques. Néanmoins, l'optimisation des cohortes dans le document repose largement sur l'expertise du praticien ou des codes de diagnostic connus.

Il existe toujours un besoin de fournir des méthodes d'analyse biologique plus précises et/ou permettant de détecter plus précocement un ou des paramètres biologiques dont les valeurs correspondent à un état de santé anormal ou potentiellement anormal.

### Description

De manière surprenante, l'Inventeur a mis en évidence qu'un paramètre biologique peut être en dérive ou en potentielle dérive, i.e. correspondre à un état de santé anormal ou potentiellement anormal pour ce paramètre biologique, alors même que la valeur de ce paramètre biologique est comprise à l'intérieur de la plage de valeurs de référence pour ce paramètre biologique.

En particulier, dans le cadre de la présente invention, un paramètre biologique qui ne varie pas dans le temps autour d'une valeur moyenne de référence est considéré comme en dérive ou en potentielle dérive, quand bien même sa valeur est comprise dans la plage de valeurs de référence. Ainsi, la présente invention permet de détecter des paramètres biologiques correspondant à un état clinique anormal ou potentiellement anormal, mais qui n'auraient pas été considérés comme tels en comparant leurs valeurs par rapport à une plage de valeurs de référence.

L'analyse de la dérive d'un paramètre biologique selon l'invention peut avantageusement être utilisée comme aide au diagnostic précoce d'une maladie, par exemple en orientant vers le diagnostic d'une maladie lors de sa phase asymptomatique.

L'analyse de la dérive d'un paramètre biologique selon l'invention permet également avantageusement de prévenir ou tout au moins retarder l'établissement d'une maladie, en permettant par exemple la mise en place d'un traitement précoce pour stopper ou ralentir la dérive dudit paramètre biologique, par exemple avant même l'apparition des premiers symptômes.

De manière avantageuse, la présente invention permet également d'orienter l'analyse médicale vers la mesure d'au moins un autre paramètre biologique statistiquement lié à un paramètre biologique détecté comme étant en dérive ou en potentielle dérive. Ledit autre paramètre biologique peut par exemple être directement corrélé à une pathologie à laquelle le praticien ne penserait pas en premier lieu, à partir de l'examen clinique et/ou d'une analyse biologique ne s'intéressant pas à la dérive métabolique.

Un premier objet de l'invention concerne une méthode d'analyse de la dérive métabolique d'au moins un paramètre biologique quantitatif chez un sujet, selon la revendication 1.

Le paramètre biologique quantitatif mesuré à l'étape a) est sélectionné dans le groupe consistant en un paramètre sérique, un paramètre infectieux, un paramètre clinique et un indicateur multi-paramètre, selon la revendication 1.

La méthode d'analyse ci-dessus est de préférence caractérisée en ce que :
- ledit paramètre biologique quantitatif est en dérive si :
   ∘ la valeur absolue de la différence entre (i) la moyenne desdites valeurs et (ii) la valeur moyenne de référence est supérieure ou égale à 1 écart-type de référence, ou
   ∘ la valeur absolue de la différence entre (i) la moyenne desdites valeurs et (ii) la valeur moyenne de référence est supérieure ou égale à 0,5 écart-type de référence et inférieure à 1 écart-type de référence et la valeur absolue de la différence entre (i) au moins une desdites valeurs et (ii) la valeur moyenne de référence est supérieure ou égale à 2 écart-type de référence, ou
   ∘ au moins une desdites valeurs est supérieure ou égale à une valeur seuil maximale de référence et/ou au moins une desdites valeurs est inférieure ou égale à une valeur seuil minimale de référence,
      et/ou
- ledit paramètre biologique quantitatif est en potentielle dérive si :
   ∘ la valeur absolue de la différence entre (i) au moins une desdites valeurs ou la moyenne desdites valeurs et (ii) la valeur moyenne de référence est supérieure ou égale à 0,5 écart-type de référence et inférieure à 1 écart-type de référence, et
   ∘ si la valeur absolue de la différence entre (i) chacune desdites valeurs et (ii) la valeur moyenne de référence est inférieure à 2 écart-type de référence.

Lorsque ledit paramètre biologique quantitatif est en potentielle dérive ou en dérive, la méthode telle que définie ci-dessus comprend de préférence une étape ultérieure de mesure de la valeur dudit paramètre biologique quantitatif à un autre instant tᵢ.

Lorsque le paramètre biologique quantitatif est en dérive ou en potentielle dérive, la méthode telle que définie ci-dessus comprend de préférence les étapes suivantes : mesurer la pente d'une droite d'ajustement passant par les valeurs fournies à l'étape a) et en déduire si le paramètre en dérive ou en potentielle dérive est stable, en aggravation ou en amélioration.

Lorsque ledit paramètre biologique quantitatif est en potentielle dérive ou en dérive, la méthode telle que définie ci-dessus comprend au moins une des étapes ultérieures suivantes:
- fournir la valeur d'au moins un autre paramètre biologique statistiquement lié au paramètre biologique en potentielle dérive ou en dérive, et/ou
- mettre en œuvre une méthode de diagnostic d'une maladie ou du risque de souffrir d'une maladie, ladite maladie étant associée audit paramètre biologique en potentielle dérive ou en dérive.

La valeur moyenne de référence et/ou l'écart-type de référence est définie selon la méthode pour définir une valeur moyenne de référence et un écart-type de référence optimisés d'un paramètre biologique X telle que définie ci-dessous.

Un autre objet de l'invention concerne une méthode pour définir une valeur moyenne de référence et un écart-type de référence optimisés d'un paramètre biologique X, selon la revendication 4.

Il est également décrit une méthode d'évaluation de l'état de dérive d'un sujet et/ou d'aide au diagnostic d'une maladie ou à l'évaluation du risque de souffrir d'une maladie chez un sujet, comprenant la mise en œuvre de la méthode d'analyse de la dérive métabolique d'un sujet telle que définie ci-dessus.

Un autre objet de l'invention concerne une méthode pour optimiser une cohorte de sujets en vue de l'étude d'un paramètre biologique X, selon la revendication 6.

Il est également décrit une méthode d'évaluation de l'état de dérive d'un sujet et/ou d'aide au diagnostic d'une maladie ou à l'évaluation du risque de souffrir d'une maladie chez un sujet, comprenant :
a) mesurer la valeur d'au moins un paramètre biologique à au moins un instant t1 dans un échantillon biologique du sujet, pour obtenir au moins une valeur v1,
b) comparer la ou les valeurs obtenues à l'étape a) à une valeur seuil de référence et/ou déterminer si les valeurs obtenues à l'étape a) fluctuent autour d'une valeur moyenne de référence, et
c) si la ou une des valeurs dudit paramètre biologique est supérieure à une valeur seuil maximale de référence ou inférieure à une valeur seuil minimale de référence et/ou si lesdites valeurs ne fluctuent pas autour d'une valeur moyenne de référence, fournir la valeur d'au moins un autre paramètre biologique statistiquement lié audit paramètre biologique et/ou en déduire si le sujet souffre ou est susceptible de souffrir de la maladie.

Ledit paramètre biologique peut par exemple être le volume plaquettaire moyen, ledit paramètre biologique statistiquement lié la thyroxine libre et ladite maladie l'hypothyroïdie et/ou une maladie de Basedow.

Un autre objet de l'invention concerne un programme d'ordinateur comprenant des instructions de code de programme pour l'exécution des étapes des méthodes telles que définies ci-dessus, lorsque ledit programme est exécuté sur ordinateur.

### Paramètre biologique

Par « paramètre biologique », on désigne ici tout paramètre, quantitatif ou qualitatif, permettant, directement ou indirectement, d'évaluer l'état de santé d'un sujet.

Le paramètre biologique est sélectionné dans le groupe consistant un paramètre sérique, un paramètre infectieux, un paramètre génétique, un paramètre non sérique (tel qu'un paramètre clinique ou un paramètre lié au mode de vie) et un indicateur multi-paramètre.

Le paramètre qualitatif peut être un paramètre qualitatif fixe dans le temps (par exemple le sexe ou la présence d'un gène donné) ou variable au cours du temps.

Les paramètres génétique et lié au mode de vie sont, de préférence, des paramètres qualitatifs.

Les paramètres sériques, cliniques, non sériques et infectieux peuvent êtres des paramètres quantitatifs ou qualitatifs.

Le paramètre biologique, en particulier le paramètre sérique, infectieux, génétique et certains paramètres non sériques, peut être mesuré dans un échantillon biologique d'un sujet.

L'échantillon biologique peut être un échantillon de sang, échantillon d'urine, échantillon de liquide céphalo-rachidien, échantillon de selles ou une biopsie.

L'échantillon biologique peut avoir subi une ou plusieurs étapes de traitement préalables avant l'analyse (telles que purification, centrifugation, filtration, précipitation, PCR et/ou RT-PCT).

Par « paramètre sérique », on désigne ici un paramètre biologique mesuré dans un échantillon de sang ou un échantillon obtenu à partir d'un échantillon de sang (par exemple, dans du plasma).

Le paramètre sérique peut, par exemple, être sélectionné dans le groupe consistant en :
- le nombre, la concentration et/ou la proportion de cellules sanguines ou de populations de cellules sanguines données : par exemple, le nombre, la concentration et/ou la proportion d'hématies (appelées également érythrocytes ou globules rouges), leucocytes (appelés également globules blancs), granulocytes, poly-neutrophiles (appelés également polynucléaires neutrophiles ou granulocytes neutrophiles), poly-éosinophiles (appelés également polynucléaire éosinophiles), poly-basophiles (appelés également polynucléaire basophiles), monocytes, lymphocytes ou plaquettes,
- la numération formule sanguine (NFS),
- la concentration d'hémoglobine,
- l'hématocrite, i.e. le volume occupé par les hématies dans un volume donné du sang total,
- le thrombocrite,
- la vitesse de sédimentation,
- *VGM* (*Volume Globulaire Moyen*),
- CGMH (*Concentration Globulaire Moyenne en Hémoglobine*),
- TCMH (*Teneur Corpusculaire Moyenne en Hémoglobine*),
- la quantité, concentration et/ou proportion en une enzyme donnée, par exemple la lipasémie, la quantité, concentration et/ou proportion de Phosphatase Alcaline, Gamma GT, transaminase TgO ou transaminase TgP,
- la quantité, concentration et/ou proportion de lipides, en particulier cholestérol total, cholestérol HDL, cholestérol LDL ou triglycérides,
- la glycémie,
- la quantité, concentration et/ou proportion de chlore, acide urique, urée, ou créatinine,
- une électrophorèse des protides, en particulier la quantité, concentration et/ou proportion en Protides totaux, albumine, alpha 1 globuline, alpha 2 globuline, beta 1 globuline, beta 2 globuline et/ou gamma globuline,
- le profil protéique , en particulier la quantité, concentration et/ou proportion en protéines sériques, telles que IgA, IgG, IgM et/ou IgE,
- un ionogramme, en particulier la quantité, concentration et/ou proportion en un ou plusieurs ions, tels que sodium, potassium, calcium, magnésium, chlore, et/ou bicarbonate, par exemple dans le plasma sanguin ,
- la quantité, concentration et/ou proportion en une ou plusieurs hormones
- la quantité, concentration et/ou proportion en un antigène, par exemple en PSA (antigène prostatique) libre et/ou en PSA conjugué, ou
- le groupe sanguin (par exemple le groupe sanguin ABO).

Le paramètre infectieux est lié à la présence ou non d'un pathogène chez un sujet.

La quantité, concentration et/ou proportion du pathogène peut être importante pour déterminer si le sujet souffre d'une infection par ledit pathogène.

Lorsque le paramètre infectieux est qualitatif, il prend par exemple la valeur « infection », « absence d'infection » ou « infection possible » par un pathogène donné.

Le pathogène peut être sélectionné dans le groupe consistant en un virus, une bactérie, un champignon, un protozoaire et un parasite.

Le virus est par exemple le virus de la mononucléose, un cytomégalovirus, un virus de l'herpès ou le VIH.

La bactérie est par exemple *Helicobacter* (de préférence *Helicobacter pylori*)*.*

Le champignon est, par exemple, *Candida* (de préférence, *Candida albicans, Candida dubliniensis, Candida glabrata, Candida guilliermondii, Candida kefyr, Candida krusei, Candida lusitaniae, Candida parapsilosis* et/ou *Candida tropicalis*).

Par « paramètre génétique », on désigne ici un paramètre qualitatif lié au génome du sujet, par exemple lié à la présence d'un gène donné ou d'un groupe de gènes donné, d'une mutation, d'un variant et/ou d'un polymorphisme donné.

Le paramètre génétique est, par exemple, sélectionné dans le groupe consistant en le groupe HLA, la mutation du gène HFE entraînant l'hémochromatose et la mutation du gène CFTR entraînant la mucoviscidose.

Le paramètre non sérique désigne ici tout paramètre qui n'est pas un paramètre sérique, infectieux ou génétique.

Le paramètre non sérique est de préférence un paramètre clinique ou un paramètre lié au mode de vie.

Par « paramètre clinique », on désigne ici notamment un paramètre quantitatif ou qualitatif qui n'est pas mesuré dans un échantillon biologique.

Le paramètre clinique est, par exemple, sélectionné dans le groupe consistant en la tension (par exemple, tension systolique et/ou tension diastolique), la taille, le poids, l'âge, le tour de taille, le sexe (en particulier, masculin ou féminin), saison de naissance, une pathologie (par exemple, un cancer, un diabète, une anémie, une dépression).

Le paramètre lié au mode de vie est par exemple sélectionné dans le groupe consistant en la consommation de cigarettes, le régime alimentaire (par exemple régime sans gluten, régime sans produit laitier, régime végétarien et/ou régime végétalien), la sédentarité, le lieu de vie (par exemple nord ou sud de la France), l'ensoleillement et le travail de jour ou de nuit.

Un indicateur multi-paramètre est le résultat d'une équation mathématique comprenant au moins un paramètre biologique quantitatif comme variable, par exemple au moins deux paramètres biologiques quantitatifs. L'indicateur multi-paramètre peut par exemple comprendre ou consister en un produit, un rapport, une somme, une soustraction, une moyenne et/ou une équation impliquant cos, sin, tan et/ou exp, et impliquant notamment au moins un paramètre biologique quantitatif.

L'indicateur multi-paramètre est donc obtenu à partir d'une ou de valeurs d'au moins un paramètre biologique quantitatif qui peut notamment être mesuré dans un échantillon biologique d'un sujet.

Des exemples non limitatifs de marqueur multi-paramètre sont :
- le nombre de globules blancs total divisé par le nombre de globules rouges.
- la quantité ou concentration de cholestérol HDL divisée par la quantité ou concentration de cholestérol LDL,
- la quantité ou concentration de PSA (antigène prostatique) libre divisée par la quantité ou concentration de PSA conjugué, ou
- le nombre de lymphocytes divisé par le nombre de polynucléaires neutrophiles.

Par « paramètre biologique X statistiquement lié à un paramètre biologique Y », on désigne ici deux paramètres biologiques qui sont statistiquement liés de manière significative.

Par exemple, deux variables quantitatives sont statistiquement liées si elles tendent à varier l'une en fonction de l'autre.

La significativité statistique entre deux paramètres biologiques peut être montrée par toute méthode statistique bien connue de l'homme du métier, telle que par exemple la régression linéaire, polynomiale, logarithmique ou non-linéaire, l'analyse de la variance (ANOVA), test de Student, test du khi-carré, tri croisé, modèle linéaire général et/ou les moindres carrés partiels.

Pour évaluer si deux paramètres biologiques quantitatifs sont statistiquement liés, la méthode statistique peut par exemple être une régression linéaire, polynomiale, logarithmique ou non linéaire.

Pour évaluer si un paramètre biologique quantitatif et un paramètre biologique qualitatif sont statistiquement liés, la méthode statistique peut par exemple être une ANOVA ou un modèle linéaire général.

Pour évaluer si deux paramètres biologiques qualitatifs sont statistiquement liés, la méthode statistique peut par exemple être un tri-croisé ou un Khi-carré.

De préférence, deux paramètres biologiques sont statistiquement liés si le niveau de significativité statistique est supérieur ou égal à 90%, de préférence supérieur ou égal à 95%, plus préférentiellement supérieur ou égal à 99,5 %, par exemple supérieur ou égal à 99,9%. La significativité statistique correspond à la probabilité que le résultat obtenu ne soit pas le fruit du hasard.

### Sujet

Le terme « sujet » désigne ici un sujet humain ou un sujet animal non humain.

Le sujet animal non humain est, de préférence, un mammifère, par exemple un chat, chien, rongeur, primate, équidé, bovin, ovin, caprin.

Alternativement, le sujet animal non humain n'est pas un mammifère.

De préférence, le sujet est un sujet humain, appelé également « individu ».

Le sujet humain peut-être de tout sexe, par exemple un homme ou une femme, de tout âge, par exemple un nourrisson, enfant en bas-âge, enfant, adolescent, adulte ou personne âgée.

Le sujet peut être un sujet sain, paraître un sujet sain, présenter au moins un symptôme d'une maladie, avoir au moins un paramètre biologique non compris dans une plage de valeur de référence de ce paramètre et/ou être susceptible de souffrir d'une maladie.

### Valeur moyenne, écart-type, valeur seuil et plage de référence

Par « valeur moyenne de référence », on désigne de préférence la moyenne des valeurs d'un paramètre biologique donné dans une population de référence.

Alternativement, la valeur moyenne de référence peut être une valeur fixée, par exemple par une administration, cette valeur ne correspondant pas nécessairement à la moyenne des valeurs d'un paramètre biologique donné dans une population de référence.

Par « écart-type de référence », on désigne ici la racine carrée de la variance des valeurs d'un paramètre biologique donné dans une population de référence.

Par « population de référence », appelée également « groupe de référence », on désigne une population d'individus dans laquelle le paramètre biologique d'intérêt est mesuré.

Dans un mode de réalisation particulier, la population de référence peut par exemple être constituée d'individus sains, c'est-à-dire présentant un bon état de santé général.

La population de référence comprend de préférence au moins 50 sujets, de préférence au moins 60 sujets, plus préférentiellement au moins 70 sujets, au moins 80 sujets, au moins 90 sujets, plus préférentiellement encore au moins 100 sujets, au moins 110 sujets, par exemple au moins 120 sujets.

Par « valeur seuil de référence », on désigne ici une valeur de référence correspondant à un seuil de tolérance. Ainsi, un paramètre biologique dont la valeur est supérieure ou égale à une valeur seuil maximale de référence ou bien qui est inférieure à une valeur seuil minimale de référence est ainsi considérée comme hors tolérance. Les valeurs seuils de référence définissent des limites au-delà desquelles une action médicale doit normalement être mise en œuvre.

Les valeurs seuils de référence sont généralement définies par rapport à la valeur moyenne de référence. Dans un mode de réalisation avantageux, la valeur seuil minimale de référence peut être égale à - [n x l'écart-type de référence] et/ou la valeur seuil maximale de référence peut être égale à + [n x l'écart-type de référence]. Par exemple, la valeur seuil minimale de référence peut être égale à - 2 écart-type de référence et/ou la valeur seuil maximale de référence peut être égale à + 2 écart-type de référence. Il peut également s'agir de valeurs plus ou moins arbitraires, par exemple définies par une administration, par exemple à la suite d'accords professionnels forts.

En général, une valeur seuil de référence égale à + ou - 2 écart-type correspond à un seuil d'alerte correspondant à un état probablement pathologique, c'est-à-dire au-delà duquel une valeur est considérée comme correspondant à un état probablement pathologique.

Par « plage de valeurs de référence », appelée également « intervalle de référence », on désigne la plage comprise entre la valeur seuil minimale de référence à la valeur seuil maximale de référence, ces valeurs seuils étant comprises dans la plage.

### Méthode d'analyse de la dérive métabolique d'au moins un paramètre biologique chez un sujet

Un premier objet de l'invention est donc une méthode d'analyse de la dérive métabolique d'au moins un paramètre biologique chez un sujet.

Par l'expression « dérive métabolique » ou par le terme « dérive » associé à un paramètre biologique, on désigne ici le fait que les valeurs de ce paramètre biologique mesurées au cours du temps ne fluctuent pas autour d'une moyenne de référence.

En effet, pour un paramètre biologique dit « normal », les valeurs fluctuent au cours du temps autour d'une valeur moyenne de référence , c'est-à-dire que la moyenne des valeurs de ce paramètre biologique mesurées au cours du temps est égale ou équivalente à la valeur moyenne de référence, ladite valeur moyenne de référence étant de préférence une valeur moyenne de référence optimisée, en particulier obtenue par la méthode telle que définie ci-dessous. A partir du moment où les valeurs du paramètre biologique mesurées au cours du temps ne fluctuent plus autour d'une moyenne de référence, les fluctuations ne sont plus de l'ordre de l'aléatoire, mais traduisent une dérive ou un dérive potentielle du paramètre biologique.

Une moyenne de valeurs considérée équivalente à la valeur moyenne de référence est par exemple strictement supérieure à [« la valeur moyenne de référence » - 0,5 x « l'écart-type de référence »] et strictement inférieure à [« la valeur moyenne de référence » + 0,5 x « l'écart-type de référence »].

Par exemple, si la valeur moyenne de référence est égale à 10 et l'écart-type de référence à 0,2, alors une valeur différente de 10 qui est comprise de 9,9 à 10,1 est équivalente à la valeur moyenne de référence.

Les termes « paramètre biologique » et « sujet » sont notamment tels que définis ci-dessus dans les sections du même nom.

La présente invention a particulièrement pour objet une méthode d'analyse de la dérive métabolique d'au moins un paramètre biologique quantitatif chez un sujet, telle que définie dans la revendication 1.

La méthode d'analyse de la dérive métabolique est, de préférence, mise en œuvre par un ordinateur ou au moins partiellement mise en œuvre par un ordinateur.

La méthode d'analyse de la dérive métabolique est, de préférence, une méthode *in vitro* et/ou *ex vivo.*

L'étape a) consiste à fournir les valeurs d'au moins un paramètre biologique quantitatif mesurées à au moins deux instants t1 et t2 étalés dans le temps, pour obtenir au moins deux valeurs v1 et v2.

La méthode d'analyse de la dérive métabolique telle que définie ci-dessus peut donc comprendre ou non une étape préalable de mesure dudit paramètre biologique quantitatif dans un échantillon biologique, en particulier à au moins deux instants t1 et t2. Les mesures effectuées au cours du temps sont donc réalisées dans des échantillons biologiques du même sujet prélevés à des instants différents.

Les échantillons biologiques dans lesquels est mesuré le paramètre biologique au cours du temps sont de préférence de même nature et/ou sont de préférence prélevés et/ou mesurés dans les mêmes conditions.

Dans un mode de réalisation avantageux, l'étape a) consiste à fournir les valeurs d'au moins un paramètre biologique quantitatif mesurées à au moins trois instants t1, t2 et t3 étalés dans le temps, pour obtenir au moins trois valeurs v1, v2 et v3, par exemple à au moins 4 instants t1, t2, t3 et t4, pour obtenir au moins quatre valeurs v1, v2, v3 et v4.

Plus généralement, l'étape a) peut consister à fournir les valeurs d'au moins un paramètre biologique quantitatif mesurées à i instants t1 à ti étalés dans le temps, pour obtenir i valeurs v1 à vi, i étant un nombre entier positif supérieur ou égale à 2.

Par « étalés dans le temps » ou « séparés dans le temps », on entend que les mesures du paramètre biologique sont espacées dans le temps, de préférence d'au moins 24h. La fréquence des mesures dépend notamment du paramètre biologique considéré.

Le paramètre biologique peut par exemple être mesuré en moyenne toutes les semaines, toutes les deux semaines, toutes les trois semaines, tous les mois, tous les deux mois, tous les trois mois, tous les six mois ou encore une fois par an.

Le paramètre biologique quantitatif mesuré à l'étape a) est, sélectionné dans le groupe consistant en un paramètre sérique, un paramètre infectieux, un paramètre clinique et un indicateur multi-paramètre, tels que définis en revendication 1. Ces différents paramètres sont notamment tels que définis ci-dessus dans la section « paramètre biologique ».

L'étape b) consiste à déterminer si lesdites valeurs fluctuent autour d'une valeur moyenne de référence pour ledit paramètre biologique quantitatif.

La valeur moyenne de référence est notamment telle que définie ci-dessus.

Dans un mode de réalisation préféré, la valeur moyenne de référence est obtenue par la méthode telle que définie ci-dessous pour définir une valeur moyenne de référence optimisée et un écart-type de référence optimisé d'un paramètre biologique X appropriés pour l'analyse de la dérive métabolique.

La fluctuation des valeurs autour d'une valeur moyenne de référence peut être évaluée visuellement, par exemple au moyen d'un graphique représentant les valeurs mesurées pour ledit paramètre biologique en fonction du temps. Si les valeurs obtenues ont une répartition aléatoire ou sensiblement aléatoire autour de la valeur moyenne de référence, en particulier avec des valeurs alternativement au-dessus et en-dessous de la valeur moyenne de référence, lesdites valeurs fluctuent autour de la valeur moyenne de référence.

Au contraire, si lesdites valeurs sont systématiquement supérieures ou majoritairement supérieures à la valeur moyenne de référence ou, inversement, systématiquement inférieures ou majoritairement inférieures à la moyenne de référence, lesdites valeurs ne fluctuent pas autour de la moyenne de référence.

Alternativement, la fluctuation des valeurs autour d'une valeur moyenne de référence peut être évaluée en comparant :
- la valeur absolue de la différence entre (i) la moyenne desdites valeurs et (ii) la valeur moyenne de référence, et/ou
- la valeur absolue de la différence entre (i) au moins une desdites valeurs, de préférence chacune desdites valeurs, et (ii) la valeur moyenne de référence,
à au moins une valeur égale à [n x l'écart-type de référence], n étant un nombre positif supérieur ou égal à 0,25, de préférence supérieur ou égal à 0,5, par exemple égal à 0,5 ou 1 ou 1,5 ou 2.

Afin d'affiner l'analyse de la dérive, la fluctuation des valeurs autour d'une valeur moyenne de référence peut être évaluée en comparant :
- la différence entre (i) la moyenne desdites valeurs et (ii) la valeur moyenne de référence, et/ou
- la différence entre (i) au moins une desdites valeurs, de préférence chacune desdites valeurs, et (ii) la valeur moyenne de référence,
à au moins une valeur égale à [n x l'écart-type de référence] et/ou à au moins une valeur égale à -[n x l'écart-type de référence], n étant un nombre positif supérieur ou égal à 0,25, de préférence supérieur ou égal à 0,5, par exemple égal à 0,5 ou 1 ou 1,5 ou 2.

Une valeur égale à [n x l'écart-type de référence] préférée selon l'invention est égale à 0,5 écart-type de référence, 1 écart-type de référence, 1,5 écart-type de référence ou 2 écart-type de référence.

La valeur moyenne de référence et l'écart-type de référence sont notamment tels que définis ci-dessus dans la section « *Valeur moyenne, écart-type, valeur seuil et plage de référence* »*.*

Dans un mode de réalisation préféré de l'invention, la valeur moyenne de référence et l'écart-type de référence sont donc des valeurs optimisées pour l'analyse de la dérive, par exemple obtenues par la méthode telle que définie ci-dessous pour définir une valeur moyenne de référence et un écart-type de référence d'un paramètre biologique X appropriés pour l'analyse de la dérive métabolique.

Dans l'étape c), ledit paramètre biologique est considéré comme en dérive ou en potentielle dérive si lesdites valeurs ne fluctuent pas autour de ladite valeur moyenne de référence.

Lorsque les valeurs mesurées fluctuent autour de ladite valeur moyenne de référence pendant une ou plusieurs périodes et qu'elles ne fluctuent pas autour de ladite valeur moyenne de référence pendant une ou plusieurs périodes, l'état de dérive du paramètre biologique est donné en fonction du temps. En d'autres termes, il est indiqué si ledit paramètre biologique est considéré ou non comme en dérive ou en potentielle dérive, pour chaque période de temps au cours de laquelle la fluctuation diffère.

L'état de dérive est notamment tel que défini ci-après.

Comme expliqué ci-après, il est également possible de distinguer des sous-états de dérive et donc d'indiquer à l'étape c) les différents états de dérive et/ou sous-états de dérive du paramètre biologique successifs au cours du temps.

Dans un mode de réalisation de l'invention,
- si la valeur absolue de la différence entre (i) la moyenne desdites valeurs et (ii) la valeur moyenne de référence est supérieure ou égale à [n x l'écart-type de référence] et/ou
- si la valeur absolue de la différence entre (i) au moins une desdites valeurs, de préférence chacune desdites valeurs, et (ii) la valeur moyenne de référence est supérieure ou égale à [n x l'écart-type de référence],
en déduire que lesdites valeurs ne fluctuent pas autour de ladite valeur moyenne de référence, en particulier si n est supérieur ou égal à 0,5.

Dans un mode de réalisation avantageux de l'invention,
- si la différence entre (i) la moyenne desdites valeurs et (ii) la valeur moyenne de référence est supérieure ou égale à [n x l'écart-type de référence] ou est inférieure ou égale à -[n x l'écart-type de référence], et/ou
- si la différence entre (i) au moins une desdites valeurs, de préférence chacune desdites valeurs, et (ii) la valeur moyenne de référence est supérieure ou égale à [n x l'écart-type de référence] ou est inférieure ou égale à -[n x l'écart-type de référence],
en déduire que lesdites valeurs ne fluctuent pas autour de ladite valeur moyenne de référence, en particulier si n est supérieur ou égal à 0,5.

Dans un mode de réalisation préféré, la méthode telle que définie ci-dessus est caractérisée en ce que :
- ledit paramètre biologique quantitatif est en dérive si :
   ∘ la valeur absolue de la différence entre (i) la moyenne desdites valeurs et (ii) la valeur moyenne de référence est supérieure ou égale à 1 écart-type de référence, ou
   ∘ la valeur absolue de la différence entre (i) la moyenne desdites valeurs et (ii) la valeur moyenne de référence est supérieure ou égale à 0,5 écart-type de référence et inférieure à 1 écart-type de référence et la valeur absolue de la différence entre (i) au moins une desdites valeurs et (ii) la valeur moyenne de référence est supérieure ou égale à 2 écart-type de référence, ou
   ∘ au moins une desdites valeurs est supérieure ou égale à une valeur seuil maximale de référence et/ou au moins une desdites valeurs est inférieure ou égale à une valeur seuil minimale de référence, et/ou
- ledit paramètre biologique quantitatif est en potentielle dérive si :
   ∘ la valeur absolue de la différence entre (i) au moins une desdites valeurs ou la moyenne desdites valeurs et (ii) la valeur moyenne de référence est supérieure ou égale à 0,5 écart-type de référence et inférieure à 1 écart-type de référence, et
   ∘ si la valeur absolue de la différence entre (i) chacune desdites valeurs et (ii) la valeur moyenne de référence est inférieure à 2 écart-type de référence.

La valeur seuil minimale ou maximale de référence est notamment telle que définie ci-dessus dans la section « *Valeur moyenne, écart-type, valeur seuil et plage de référence* »*.*

Dans un mode de réalisation avantageux de l'invention, la méthode telle que définie ci-dessus est caractérisée en ce que, lorsque ledit paramètre biologique quantitatif est en potentielle dérive ou en dérive, la méthode telle que définie ci-dessus comprend une étape ultérieure de mesure de la valeur dudit paramètre biologique quantitatif à un autre instant tᵢ.

Cette étape peut permettre par exemple de confirmer une potentielle dérive et/ou suivre l'évolution d'un paramètre en dérive ou en potentielle dérive, notamment après mise en place d'un traitement et/ou en cours du traitement visant à corriger ledit paramètre biologique, i.e. visant à faire cesser la dérive ou la dérive potentielle dudit paramètre biologique.

L'autre instant tᵢ est, de préférence, situé au moins une semaine, de préférence au moins deux semaines, plus préférentiellement encore au moins trois semaines, par exemple trois semaines ou quatre semaines après la dernière valeur mesurée. L'autre instant tᵢ peut également être effectué un mois, deux mois, trois mois, six mois après.

De préférence, au moins tant que le paramètre biologique est en dérive ou en potentielle dérive, le paramètre biologique est mesuré régulièrement au cours du temps, par exemple en moyenne toutes les semaines, toutes les deux semaines, toutes les trois semaines, tous les mois, tous les deux mois, tous les trois mois, tous les 6 mois ou encore une fois par an.

Dans un mode de réalisation avantageux de l'invention, la méthode telle que définie ci-dessus est caractérisée en ce que, lorsque le paramètre biologique quantitatif est en dérive ou en potentielle dérive, la méthode telle que définie ci-dessus comprend les étapes suivantes : mesurer la pente d'une droite d'ajustement passant par les valeurs fournies à l'étape a) et en déduire si le paramètre en dérive ou en potentielle dérive est stable, en aggravation ou en amélioration.

Le paramètre en dérive ou en potentielle dérive est par exemple en aggravation si la pente de la droite d'ajustement est telle que les valeurs obtenues au cours du temps s'écartent de la valeur moyenne de référence.

Le paramètre en dérive ou en potentielle dérive est par exemple stable si la pente de la droite d'ajustement est nulle.

Le paramètre en dérive ou en potentielle dérive est par exemple en amélioration si la pente de la droite d'ajustement est telle que les valeurs obtenues au cours du temps se rapprochent de la valeur moyenne de référence.

La droite d'ajustement est obtenue par toute méthode appropriée bien connue de l'homme du métier. La droite d'ajustement est, de préférence, obtenue par un modèle de régression linéaire, par exemple l'ajustement affine, la méthode des moindres carrés, le maximum de vraisemblance et/ou l'inférence bayésienne.

Il est possible de définir différents états ou sous-états de dérive en fonction des valeurs obtenues. Il est clair pour l'homme du métier que l'invention n'est nullement limitée aux états et/ou sous-états de dérive ou de dérive potentielle qui sont données ici à titre d'exemples.

A titre d'exemple, il est possible de distinguer les états suivants : absence de dérive, dérive et dérive potentielle.

L'état correspondant à l'absence de dérive peut par exemple comprendre les sous-états « excellent » ou « bon ».

L'état de dérive peut par exemple comprendre les sous-états « dérive » et « dérive importante ».

L'état ou sous-état de dérive ou de potentielle dérive peut être qualifiée de « basse » si la moyenne des valeurs est inférieure à la valeur moyenne de référence et de « haute » si la moyenne des valeurs est supérieure à la valeur moyenne de référence.

Un paramètre biologique peut par exemple être classé en état excellent lorsque la valeur absolue de la différence entre (i) la moyenne des valeurs et (ii) la valeur moyenne de référence est strictement inférieure à 0,5 écart-type et que la valeur absolue de la différence entre (i) la valeur la plus récente et (ii) la valeur moyenne de référence est strictement inférieure 0,5 écart-type.

Un paramètre biologique peut par exemple être classé en état bon lorsque :
- la valeur absolue de la différence entre (i) la moyenne des valeurs et (ii) la valeur moyenne de référence est strictement inférieure à 0,5 écart-type et la différence entre (i) la valeur la plus récente et (ii) la valeur moyenne de référence est supérieure ou égale 0,5 écart-type,
- la valeur absolue de la différence entre (i) la moyenne des valeurs et (ii) la valeur moyenne de référence est strictement inférieure à 0,5 écart-type et la différence entre (i) la valeur la plus récente et (ii) la valeur moyenne de référence est inférieure ou égale -0,5 écart-type,
- la différence entre (i) la moyenne des valeurs et (ii) la valeur moyenne de référence est comprise de 0,5 écart-type à 1 écart-type et la différence entre (i) la valeur la plus récente et (ii) la valeur moyenne de référence est strictement inférieure à 0,5 écart-type,
- la différence entre (i) la moyenne des valeurs et (ii) la valeur moyenne de référence est comprise de -1 écart-type à -0,5 écart-type et la différence entre (i) la valeur la plus récente et (ii) la valeur moyenne de référence est strictement supérieure à -0,5 écart-type,
- la différence entre (i) la moyenne des valeurs et (ii) la valeur moyenne de référence est comprise de 0,5 écart-type à 1 écart-type et la différence entre (i) au moins une des valeurs et (ii) la valeur moyenne de référence est strictement supérieure à 0,5 écart-type, ou
- la valeur absolue de la différence entre (i) la moyenne des valeurs et (ii) la valeur moyenne de référence est comprise de -1 écart-type à -0,5 écart-type et la différence entre (i) au moins une des valeurs et (ii) la valeur moyenne de référence est strictement inférieure à -0,5 écart-type.

Un paramètre biologique est par exemple classé en dérive potentielle lorsque :
- la différence entre (i) la moyenne des valeurs et (ii) la valeur moyenne de référence est supérieure ou égale à 0,5 écart-type de référence et est inférieure à 1 écart-type de référence et la différence entre (i) chacune des valeurs et (ii) la valeur moyenne de référence est supérieure ou égale à 0,5 écart-type de référence et est inférieure à 1 écart-type de référence,
- la différence entre (i) la moyenne des valeurs et (ii) la valeur moyenne de référence est supérieure ou égale à -1 écart-type de référence et est inférieure à -0,5 écart-type de référence et la différence entre (i) chacune des valeurs et (ii) la valeur moyenne de référence est supérieure ou égale à -1 écart-type de référence et est inférieure à -0,5 écart-type de référence,
- la différence entre (i) la moyenne des valeurs et (ii) la valeur moyenne de référence est supérieure ou égale à 1 écart-type de référence et est inférieure à 2 écart-type de référence et la différence entre (i) la valeur la plus récente et (ii) la valeur moyenne de référence est inférieure à 1 écart-type de référence, ou
- la différence entre (i) la moyenne des valeurs et (ii) la valeur moyenne de référence est supérieure ou égale à -2 écart-type de référence et est inférieure à -1 écart-type de référence et la différence entre (i) la valeur la plus récente et (ii) la valeur moyenne de référence est supérieure à - 1 écart-type de référence.

Un paramètre biologique est par exemple classé en dérive lorsque :
- la différence entre (i) la moyenne des valeurs et (ii) la valeur moyenne de référence est supérieure ou égale à 1 écart-type de référence et est inférieure à 2 écart-type de référence,
- la différence entre (i) la moyenne des valeurs et (ii) la valeur moyenne de référence est supérieure ou égale à -2 écart-type de référence et est inférieure à -1 écart-type de référence,
- la différence entre (i) la moyenne des valeurs et (ii) la valeur moyenne de référence est supérieure ou égale à 1 écart-type de référence et est inférieure à 2 écart-type de référence et la différence entre (i) chacune des valeurs et (ii) la valeur moyenne de référence est supérieure ou égale à 1 écart-type de référence et est inférieure à 2 écart-type de référence,

- la différence entre (i) la moyenne des valeurs et (ii) la valeur moyenne de référence est supérieure ou égale à -2 écart-type de référence et est inférieure à -1 écart-type de référence et la différence entre (i) chacune des valeurs et (ii) la valeur moyenne de référence est supérieure ou égale à -2 écart-type de référence et est inférieure à -1 écart-type de référence,
- la différence entre (i) la moyenne des valeurs et (ii) la valeur moyenne de référence est supérieure ou égale à 1 écart-type de référence et est inférieure à 2 écart-type de référence et la différence entre (i) la valeur la plus récente et (ii) la valeur moyenne de référence est supérieure à 2 écart-type de référence,
- la différence entre (i) la moyenne des valeurs et (ii) la valeur moyenne de référence est supérieure ou égale à -2 écart-type de référence et est inférieure à -1 écart-type de référence et la différence entre (i) la valeur la plus récente et (ii) la valeur moyenne de référence est inférieure à -2 écart-type de référence,
- la différence entre (i) la moyenne des valeurs et (ii) la valeur moyenne de référence est supérieure ou égale à 2 écart-type de référence et est inférieure à 3 écart-type de référence et la différence entre (i) la valeur la plus récente et (ii) la valeur moyenne de référence est inférieure à 2 écart-type de référence, ou
- la différence entre (i) la moyenne des valeurs et (ii) la valeur moyenne de référence est supérieure ou égale à -3 écart-type de référence et est inférieure à -2 écart-type de référence et la différence entre (i) la valeur la plus récente et (ii) la valeur moyenne de référence est supérieure à - 2 écart-type de référence.

Un paramètre biologique est par exemple classé en dérive importante lorsque :
- la différence entre (i) la moyenne des valeurs et (ii) la valeur moyenne de référence est supérieure ou égale à 2 écart-type de référence, par exemple supérieure ou égale à 3 écart-type de référence,
- la différence entre (i) la moyenne des valeurs et (ii) la valeur moyenne de référence est inférieure ou égale à -2 écart-type de référence, par exemple inférieure ou égale à -3 écart-type de référence, ou
- la valeur la plus récente est inférieure à une valeur seuil minimale de référence ou supérieure à une valeur seuil maximale de référence.

Lorsqu'au moins 3 valeurs sont fournies à l'étape a), l'étape b) de la méthode telle que définie ci-dessus comprend de préférence l'analyse de la dérive des valeurs prises deux à deux, en partant de la valeur mesurée la plus ancienne ou alternativement de la valeur mesurée la plus récente.

La méthode telle que définie ci-dessus peut alors être caractérisée en ce que :
- dans l'étape b), il est déterminé si lesdites valeurs fluctuent autour d'une valeur moyenne de référence pour ledit paramètre biologique quantitatif pour chaque période de temps comprise entre deux instants tx et tz tels que l'état de dérive entre chacune des valeurs successives prises deux en deux entre l'instant tx et tz est identique (*c'est-à-dire que l'état de dérive entre vx et vx+1, celui entre vx+1 et vx+2, celui entre vx+2 et vx+3, etc. jusqu'à celui entre vx+y-1 et vz, avec x+y=z, sont identiques),*
- dans l'étape c), pour chaque période de temps définie à l'étape b), en conclure que ledit paramètre biologique est en dérive ou en potentielle dérive si lesdites valeurs ne fluctuent pas autour de ladite valeur moyenne de référence.

L'étape b) peut ainsi avantageusement comprendre la comparaison l'état de dérive et/ou le sous-état de dérive des valeurs vi et vi+1 avec l'état de dérive et/ou le sous-état de dérive des valeurs vi+1 et vi+2 :
- si les états de dérive et/ou les sous-états de dérive sont identiques, l'étape b) peut alors comprendre la comparaison de l'état de dérive et/ou sous-état de dérive des valeurs vi, vi+1 et vi+2 avec l'état de dérive et/ou le sous-état de dérive des valeurs vi+2 et vi+3, et ainsi de suite,
- si les états de dérive et/ou les sous-états de dérive sont différents, l'étape b) peut alors comprendre la comparaison de l'état de dérive et/ou sous-état de dérive des valeurs vi+2 et vi+3 avec l'état de dérive et/ou le sous-état de dérive des valeurs vi+3 et vi+4, et ainsi de suite et l'étape c) comprendra au moins deux états ou sous-états de dérive différents du paramètre biologique différent, dont un état de dérive ou sous-état de dérive défini entre les instants vi et vi+1.

Dans l'étape c), plusieurs états de dérive et/ou sous-états de dérive différents au cours du temps du paramètre biologique peuvent dont être déterminés.

Ainsi, l'étape b) peut par exemple comprendre :
(i) déterminer l'état de dérive et/ou sous-état de dérive des valeurs v1 et v2,
(ii) déterminer l'état de dérive des valeurs v2 et v3,
(iii) si l'état de dérive et/ou sous-état de dérive est le même pour les valeurs v1 et v2 et pour les valeurs v2 et v3,
   a. déterminer l'état de dérive et/ou sous-état de dérive des valeurs v1, v 2 et v3,
   b. déterminer l'état de dérive et/ou sous-état de dérive des valeurs v3 et v4,
   c. si l'état de dérive et/ou sous-état de dérive est le même pour les valeurs v1, v2 et v3 et pour les valeurs v3 et v4, déterminer l'état de dérive et/ou sous-état de dérive des valeurs v1 à v4, et le comparer à l'état de dérive et/ou sous-état de dérive des valeurs v4 et v5, et ainsi de suite.
   d. si l'état de dérive et/ou sous-état de dérive pour les valeurs v1, v2 et v3 est différent de celui pour les valeurs v3 et v4, en conclure l'état de dérive et/ou sous-état de dérive entre les instants t1 et t3 dans l'étape c) et comparer l'état de dérive et/ou sous-état de dérive des valeurs v4 et v5 avec celui des valeurs v5 et v6, etc.,
(iv) si l'état de dérive et/ou sous-état de dérive pour les valeurs v1 et v2 est différent de celui pour les valeurs v2 et v3,
   a. en conclure l'état de dérive et/ou sous-état de dérive du paramètre biologique entre les instants t1 et t2 à partir des valeurs v1 et v2,
   b. comparer l'état de dérive et/ou sous-état de dérive des valeurs v3 et v4 et celui des valeurs v4 et v5, etc..

Les valeurs analysées prises deux à deux ont été de préférence mesurées à moins de 5 ans d'intervalle, par exemple à moins de deux ans d'intervalle.

Les valeurs analysées deux à deux sont de préférence distantes de moins de 2 écarts types.

Si les valeurs analysées deux à deux sont distantes d'au moins 2 écarts types, chacune de ses valeurs est analysée individuellement ou avec une autre valeur.

Lorsque le paramètre biologique quantitatif est en potentielle dérive ou en dérive, la méthode telle que définie ci-dessus peut comprendre une étape ultérieure consistant à fournir la valeur d'au moins un autre paramètre biologique statistiquement lié au paramètre biologique en potentielle dérive ou en dérive. De préférence, la méthode peut alors comprendre l'analyse de la dérive métabolique dudit au moins un autre paramètre biologique statistiquement lié au paramètre biologique en potentielle dérive ou en dérive, par exemple par la méthode d'analyse telle que définie ci-dessus. Alternativement, la valeur dudit autre paramètre biologique est comparée à une valeur moyenne de référence et/ou à une valeur seuil de référence.

Ledit autre paramètre biologique peut être un paramètre quantitatif (par exemple un paramètre sérique, un paramètre infectieux, un paramètre clinique ou un indicateur multi-paramètre) ou un paramètre qualitatif (par exemple, un paramètre génétique, un paramètre infectieux, un paramètre non sérique ou un paramètre lié au mode de vie).

Un paramètre biologique statistiquement lié à un paramètre biologique est notamment tel que défini ci-dessus dans la section « *Paramètre biologique* »*.*

Des exemples non limitatifs sont donnés ci-après.

La vitamine D est un exemple de paramètre biologique statistiquement lié à la Vitamine B12. L'Inventeur a en effet mis en évidence que, lorsque la concentration en vitamine B12 est en dérive basse, plus de 61% des patients présentent une carence de vitamine D, alors que lorsque la concentration en vitamine B12 n'est pas en dérive basse, seulement 44% des patients présentent une carence de vitamine D.

Ainsi, si la concentration en vitamine B12 est en dérive basse, en particulier lorsque la concentration en vitamine B12 est inférieur à 316pg/ml, la méthode telle que définie ci-dessus comprend de préférence une étape ultérieure de mesure de la concentration en vitamine D.

Si la concentration en vitamine D est en dérive, en particulier lors d'une concentration en vitamine D inférieure à 20 ng/ml, ce qui correspond à une carence en vitamine D la méthode comprend de préférence une étape ultérieure de détermination du groupe HLA. Si le patient est HLA-DQ02, HLA-DQ8, HLA-DR3 ou HLA-A1, la méthode comprend de préférence une étape d'évaluation de l'absorption intestinale ou de diagnostic de la maladie coeliaque. **En** effet, par exemple un taux inférieur à 21ng/ml est plus particulièrement spécifique à la population porteuse des gènes HLA-DQ02, HLA-DQ8, HLA-DR3 ou HLA-A1, ces mêmes gènes étant connus pour entrainer des malabsorptions intestinales, en particulier des malabsorptions de la vitamine D.

Par exemple, si le volume plaquettaire moyen est en dérive basse, la méthode comprend une étape ultérieure de mesure de la thyroxine libre.

Lorsque le paramètre biologique quantitatif et/ou un paramètre biologique statistiquement lié audit paramètre biologique est en potentielle dérive ou en dérive, la méthode telle que définie ci-dessus peut comprendre une étape ultérieure de mise en œuvre d'une méthode, de préférence *in vitro* et/ou *ex vivo,* de diagnostic d'une maladie ou du risque de souffrir d'une maladie, ladite maladie étant associée au paramètre biologique en potentielle dérive ou en dérive et/ou au paramètre biologique statistiquement lié audit paramètre biologique en potentielle dérive ou en dérive.

La méthode selon l'invention présente l'avantage de permettre un diagnostic plus précoce d'une maladie. En effet, le diagnostic peut être effectué dès qu'un paramètre biologique associé à cette maladie est en dérive ou potentielle dérive, au lieu d'attendre que ce paramètre biologique soit hors seuil de tolérance.

Lorsque ledit paramètre biologique quantitatif est en potentielle dérive ou en dérive, la méthode telle que définie ci-dessus comprend au moins une des étapes ultérieures suivantes:
- fournir la valeur d'au moins un autre paramètre biologique statistiquement lié au paramètre biologique en potentielle dérive ou en dérive, par exemple telle que définie ci-dessus, et/ou
- mettre en œuvre une méthode, de préférence *in vitro* et/ou *ex vivo,* de diagnostic d'une maladie ou du risque de souffrir d'une maladie, ladite maladie étant associée au paramètre biologique en potentielle dérive ou en dérive ou audit paramètre biologique statistiquement lié au paramètre biologique en potentielle dérive ou en dérive, par exemple telle que définie ci-dessus.

Si le paramètre biologique en dérive ou en potentielle dérive est l'indicateur multi-paramètre défini par le nombre de globules blancs total divisé par le nombre de globules rouges, la méthode comprend une étape de diagnostic ou d'évaluation du risque de souffrir d'une infection par *Candida albicans,* par exemple une candidose invasive.

Si le paramètre biologique en dérive ou en potentielle dérive est l'indicateur multi-paramètre défini par le nombre de lymphocytes divisé par le nombre de polynucléaires neutrophiles, la méthode comprend une étape de diagnostic ou d'évaluation du risque de souffrir d'une infection le Cytomégalovirus (CMV).

### Méthode pour définir une valeur moyenne de référence optimisée et un écart-type de référence optimisé d'un paramètre biologique X appropriés pour l'analyse de la dérive métabolique dudit paramètre biologique

La présente invention permet également de fournir une valeur moyenne de référence et un écart-type de référence d'un paramètre biologique X optimisés pour l'analyse de la dérive métabolique.

La méthode pour définir une valeur moyenne de référence et un écart-type de référence d'un paramètre biologique X selon l'invention prend en effet en compte la dérive ou la dérive potentielle éventuelle de paramètre(s) biologique(s) statistiquement lié(s) au paramètre biologique X pour définir un groupe optimisé, afin d'obtenir une valeur moyenne de référence et écart-type de référence plus représentatif d'un équilibre métabolique optimum.

La présente invention a particulièrement pour objet une méthode pour définir une valeur moyenne de référence et un écart-type de référence optimisés d'un paramètre biologique quantitatif X, selon la revendication 4.

Le paramètre biologique est tel que défini ci-dessus dans la section du même nom.

Le paramètre biologique est de préférence sélectionné dans le groupe consistant un paramètre sérique, un paramètre infectieux, un paramètre clinique, un paramètre génétique, un paramètre lié au mode de vie et un indicateur multi-paramètre, chacun de ces paramètres étant notamment tels que définis ci-dessus dans la section « Paramètre biologique ».

Le paramètre biologique X est de préférence un paramètre quantitatif.

L'expression « paramètre biologique en dehors d'une valeur seuil de référence » signifie qu'au moins une des valeurs et/ou la moyenne des valeurs dudit paramètre biologique est supérieure ou égale à une valeur seuil de référence maximale ou est inférieure ou égale à une valeur seuil de référence minimale.

La méthode pour définir une valeur moyenne de référence et un écart-type de référence d'un paramètre biologique X est, de préférence, mise en œuvre par un ordinateur ou au moins partiellement mise en œuvre par un ordinateur.

La méthode pour définir une valeur moyenne de référence et un écart-type de référence d'un paramètre biologique X est, de préférence, une méthode *in vitro* et/ou *ex vivo.*

L'étape a) consiste à fournir :
∘ la ou une valeur du paramètre biologique X mesurée à un instant donné et/ou des valeurs du paramètre biologique X, par exemple au moins deux valeurs, mesurées à différents instants séparés dans le temps, et
∘ la valeur d'au moins un autre paramètre biologique mesurée à un instant donné et/ou des valeurs d'au moins un autre paramètre biologique mesurées à différents instants séparés dans le temps, par exemple au moins deux valeurs, pour chaque sujet d'un groupe consistant en au moins 50 sujets.

L'étape a) comprend de préférence de fournir au moins deux valeurs d'au moins un autre paramètre biologique quantitatif mesurées à différents instants séparés dans le temps et, optionnellement, la valeur d'au moins un autre paramètre biologique qualitatif mesurée à un instant donné et/ou des valeurs d'au moins un autre paramètre biologique qualitatif mesurées à différents instants séparés dans le temps.

La méthode pour définir une valeur moyenne de référence et un écart-type de référence d'un paramètre biologique X telle que définie ci-dessus peut donc comprendre ou non une étape préalable de mesure dudit paramètre biologique X et d'au moins un autre paramètre biologique, à un instant donné ou à différents instants séparés dans le temps, par exemple dans un échantillon biologique.

Les mesures effectuées au cours du temps sont réalisées dans des échantillons biologiques du même sujet prélevés à des instants différents.

Les échantillons biologiques dans lesquels est mesuré un paramètre biologique donné au cours du temps sont de préférence de même nature et/ou sont de préférence prélevés et/ou mesurés dans les même conditions.

Par « étalés dans le temps », on entend que les mesures du paramètre biologique sont espacées dans le temps, de préférence d'au moins 24h. La fréquence des mesures dépend notamment du paramètre biologique considéré.

Le paramètre biologique peut par exemple être mesuré en moyenne toutes les semaines, toutes les deux semaines, toutes les trois semaines, tous les mois, tous les deux mois, tous les trois mois, tous les six mois ou encore une fois par an.

L'étape b) consiste à identifier le ou les paramètres biologiques statistiquement liés au paramètre biologique X à partir de ladite valeur et/ou de la moyenne desdites valeurs du paramètre biologique X et d'au moins un autre paramètre biologique.

En particulier, l'étape b) consiste à identifier le ou les paramètres biologiques quantitatifs et, optionnellement, le ou les paramètres biologiques qualitatifs, statistiquement liés au paramètre biologique quantitatif X, à partir d'une desdites valeurs et/ou de la moyenne desdites valeurs du paramètre biologique quantitatif X, d'une desdites valeurs ou de la moyenne desdites valeurs d'au moins un autre paramètre biologique quantitatif et, optionnellement, d'une desdites valeurs d'au moins un autre paramètre biologique qualitatif.

Un paramètre biologique statistiquement lié à un paramètre biologique est notamment tel que défini ci-dessus dans la section « *Paramètre biologique* »*.*

En particulier, la significativité statistique entre deux paramètres biologiques peut être montrée par toute méthode statistique bien connue de l'homme du métier, telle que par exemple corrélation linéaire, covariance, analyse de la variance (ANOVA), test de Student, test du khi-deux, Tri Croisé.

De préférence, deux paramètres biologiques sont statistiquement liés si le niveau de significativité statistique est supérieur ou égal à 90%, de préférence supérieur ou égal à 95%, plus préférentiellement supérieur ou égal à 99%, par exemple supérieur ou égal à 99,5%.

Dans l'étape c), pour chaque paramètre biologique statistiquement lié au paramètre biologique X identifié à l'étape b), sont écartés du groupe :
(i) les sujets chez lesquels ce paramètre biologique est en dehors d'une valeur seuil de référence et/ou chez lesquels ce paramètre biologique est en dérive ou potentielle dérive, lorsqu'il s'agit d'un paramètre biologique quantitatif, et/ou
(ii) les sujets chez lesquels ce paramètre biologique est influent, lorsqu'il s'agit d'un paramètre biologique qualitatif,
afin de définir un sous-groupe de sujets.

En particulier, dans l'étape c), pour chaque paramètre biologique identifié à l'étape b), sont écartés du groupe :
(i) les sujets chez lesquels ce paramètre biologique quantitatif est en dérive ou potentielle dérive et
(ii) le cas échéant, les sujets chez lesquels ce paramètre biologique qualitatif est influent,
   afin de définir un sous-groupe de sujets,

L'évaluation de la dérive ou de la potentielle dérive d'un paramètre biologique est notamment effectuée comme décrit ci-dessus dans la méthode d'analyse de la dérive métabolique d'au moins un paramètre biologique chez un sujet.

Ainsi, la méthode selon l'invention permet avantageusement d'éliminer du groupe de sujets chez lesquels un paramètre biologique corrélé au paramètre biologique X étudié est en dérive ou en potentielle dérive (grâce au (i)). La méthode selon l'invention permet ainsi d'optimiser le groupe de référence à partir duquel sont calculés la valeur moyenne de référence et l'écart-type de référence du paramètre biologique X.

Par « les sujets chez lesquels ce paramètre biologique est influent », on désigne des sujets chez lesquels ce paramètre biologique qualitatif a une valeur que l'on ne souhaite pas prendre en compte pour déterminer la moyenne de référence et l'écart-type de référence optimisés.

Des exemples non limitatifs de paramètres pouvant être influents sont le sexe, le poids, la taille et/ou le groupe sanguin.

Lorsqu'un paramètre biologique qualitatif est influent, la méthode telle que définie ci-dessus peut comprendre de définir la valeur moyenne de référence et l'écart-type de référence dans l'autre sous-groupe ou un des autres sous-groupes correspondant à une autre valeur ou une autre plage de valeurs du paramètre qualitatif, en particulier parmi les sujets écartés à l'étape c) ii). L'éventuelle étape c') et l'étape d) peuvent alors être effectuées dans chaque sous-groupe correspondant à une autre valeur ou une autre plage de valeurs du paramètre qualitatif.

Par exemple, si le sexe est un paramètre biologique influent, la moyenne de référence et l'écart-type de référence peuvent être calculés respectivement dans un sous-groupe constitué de sujets masculins et dans un sous-groupe constitué de sujets féminins.

Par exemple, le groupe sanguin est un paramètre biologique influent sur les valeurs de lipasémie. Les sujets de groupe A ont ainsi des valeurs de lipasémie globalement plus faibles que les sujets du groupe O, et donc des valeurs moyennes et des écart-types significativement différentes. La moyenne de référence optimisée et l'écart-type de référence optimisé peuvent alors être calculés respectivement dans un sous-groupe constitué de sujets de groupe A et dans un sous-groupe constitué de sujets de groupe O.

La méthode telle que définie ci-dessus peut optionnellement comprendre une étape c') consistant à fournir les valeurs d'au moins un paramètre biologique sérique mesurées à des instants séparés dans le temps, par exemple à au moins deux instants t1 et t2 étalés dans le temps, pour chaque sujet du ou des sous-groupes définis à l'étape c) et écarter du ou des sous-groupes les sujets chez lesquels ce paramètre biologique sérique est en dehors d'une valeur seuil de référence et/ou chez lesquels ce paramètre biologique est en dérive ou potentielle dérive, afin de définir un ou des deuxièmes sous-groupes de sujets.

L'étape c') peut, par exemple, permettre d'écarter du sous-groupe un ou des sujets qui seraient en train de développer une infection, une carence, ou toute autre déséquilibres, sans en présenter encore les symptômes, le paramètre biologique sérique mesuré n'étant de préférence pas statistiquement lié au paramètre biologique X.

L'étape c') permet d'optimiser encore davantage le groupe de référence à partir duquel sont calculés la valeur moyenne de référence et l'écart-type de référence du paramètre biologique X.

L'étape d) consiste à définir la moyenne et l'écart-type du paramètre biologique X dans le sous-groupe de sujets défini à l'étape c) ou dans le ou les deuxièmes sous-groupes de sujets défini à l'étape c', optionnellement en tenant compte d'au moins un paramètre biologique quantitatif.

Selon les cas, la moyenne et l'écart type de référence optimisés peuvent en effet être définis en fonction d'un ou plusieurs paramètres quantitatifs statistiquement lié au paramètre biologique X et/ou en fonction d'un ou plusieurs paramètres qualitatifs statistiquement lié au paramètre biologique X.

Des exemples non limitatifs sont la moyenne et l'écart type du poids idéal définis en fonction de la taille du sujet ou encore la moyenne et l'écart-type du taux d'hémoglobine en fonction de la taille ou le poids du sujet.

### Méthode d'optimisation d'une cohorte de sujets

La présente invention permet également de fournir une méthode d'optimisation d'une cohorte de sujets en vue d'une étude, par exemple de facteurs impliqués dans et/ou influençant une maladie ou une réponse à un traitement.

La méthode d'optimisation d'une cohorte de sujets selon l'invention prend en effet en compte la dérive ou la dérive potentielle éventuelle de paramètre(s) biologique(s) statistiquement lié(s) au paramètre biologique X faisant l'objet de l'étude pour définir une cohorte optimisée.

La présente invention a ainsi pour objet une méthode pour optimiser une cohorte de sujets en vue de l'étude d'un paramètre biologique X, selon la revendication 6.

Il est également décrit une méthode pour optimiser une cohorte de sujets en vue de l'étude d'un paramètre biologique X, comprenant les étapes de :
a) fournir (i) au moins deux valeurs mesurées à différents instants séparés dans le temps et/ou une moyenne d'au moins deux valeurs mesurées à différents instants séparés dans le temps d'au moins un paramètre biologique quantitatif statistiquement lié au paramètre biologique X et, optionnellement, (ii) une valeur mesurée à un instant donné ou au moins deux valeurs mesurées à différents instants séparées dans le temps d'au moins un paramètre biologique qualitatif statistiquement lié au paramètre biologique X, pour chaque sujet d'une cohorte de sujets,
b) pour chaque paramètre biologique quantitatif, écarter de la cohorte ou placer dans un sous-groupe les sujets chez lesquels ce paramètre biologique est en dérive ou potentielle dérive et, optionnellement, pour chaque paramètre biologique qualitatif, écarter de la cohorte ou placer dans un sous-groupe les sujets chez lesquels ce paramètre biologique est influent, afin d'obtenir une cohorte optimisée, en particulier à partir des valeurs fournies à l'étape b),
c) optionnellement, fournir la valeur mesurée à un instant donné, au moins deux valeurs mesurées à différents instants séparés dans le temps et/ou une moyenne d'au moins deux valeurs mesurées à différents instants séparés dans le temps du paramètre biologique X dans la cohorte optimisée définie à l'étape b).

Les différents termes sont tels que définis ci-dessus dans les différents sections.

Les cohortes optimisées ainsi obtenues peuvent par exemple comprendre une cohorte de sujets dont au moins un paramètre donné n'est pas en dérive ou potentielle dérive, une cohorte de sujets en possible dérive pour ledit au moins un paramètre biologique et/ou une cohorte de sujets en dérive pour ledit au moins un paramètre biologique (par exemple en dérive importante et/ou en dérive).

Au sein de la ou des cohortes, il est également possible de tenir compte des différents paramètres influents sur ces paramètres en dérive ou en dérive potentielle.

La moyenne de référence et l'écart-type de référence sont de préférence définis selon la méthode pour définir une valeur moyenne de référence et un écart-type de référence d'un paramètre biologique X optimisés, telle que définie ci-dessus dans la section du même nom.

L'évaluation de la dérive ou de la potentielle dérive d'un paramètre biologique est notamment effectuée comme décrit ci-dessus dans la méthode d'analyse de la dérive métabolique d'au moins un paramètre biologique chez un sujet.

La méthode d'optimisation d'une cohorte de sujets telle que définie ci-dessus est, de préférence, mise en œuvre par un ordinateur ou au moins partiellement mise en œuvre par un ordinateur.

La méthode d'optimisation d'une cohorte de sujets telle que définie ci-dessus est, de préférence, une méthode *in vitro* et/ou *ex vivo.*

La méthode d'optimisation d'une cohorte de sujets telle que définie ci-dessus peut donc comprendre une étape préalable de mesure dudit paramètre biologique X et d'au moins un paramètre biologique statistiquement lié audit paramètre biologique dans un échantillon biologique,

Entre les étapes a) et b), la méthode peut comprendre une étape d'analyse de la dérive d'au moins un paramètre biologique et en déduire si ce paramètre biologique est compris dans l'un des trois états suivants : absence de dérive, en dérive ou en dérive potentielle.

Il est également possible d'utiliser des sous-états.

Par exemple, un état en absence de dérive peut être excellent ou bon.

Par exemple, un état en dérive peut être en dérive ou dérive importante.

Ces états et /ou sous-états sont par exemple tels que définis ci-dessus.

Il est possible d'utiliser des sous-classes, par exemple : excellent, bon, Dans l'étape b), (i), il est possible de définir des sous-classes de dérive : par exemple, dérive potentielle, dérive, dérive importante, et hors tolérance. Les paramètres biologiques dont les valeurs sont « normales » peuvent être classées en excellent ou bon.

La méthode telle que définie ci-dessus peut comprendre des étapes ultérieures de classement des résultats de l'étude de la cohorte selon la même méthode et d'étude de l'évolution des niveaux de dérive.

### Méthode d'évaluation de l'état de dérive d'un sujet et/ou d'aide au diagnostic d'une maladie ou à l'évaluation du risque de souffrir d'une maladie chez un sujet

Il est également décrit une méthode d'évaluation de l'état de dérive d'un sujet et/ou d'aide au diagnostic d'une maladie ou à l'évaluation du risque de souffrir d'une maladie chez un sujet.

La méthode est, de préférence, mise en œuvre par un ordinateur ou au moins partiellement mise en œuvre par un ordinateur.

La méthode est, de préférence, une méthode *in vitro* et/ou *ex vivo.*

Dans un mode de réalisation préféré, cette méthode comprend la mise en œuvre d'une méthode d'analyse de la dérive métabolique d'au moins un paramètre biologique chez un sujet telle que définie ci-dessus dans la section du même nom.

Dans un autre mode de réalisation, la méthode d'évaluation de l'état de dérive d'un sujet et/ou d'aide au diagnostic d'une maladie ou à l'évaluation du risque de souffrir d'une maladie chez un sujet, comprend les étapes suivantes:
a) mesurer la valeur d'au moins un paramètre biologique à au moins un instant t1 dans un échantillon biologique du sujet, pour obtenir au moins une valeur v1,
b) comparer la ou les valeurs obtenues à l'étape a) à une valeur seuil de référence et/ou déterminer si les valeurs obtenues à l'étape a) fluctuent autour d'une valeur moyenne de référence, et
c) si la ou une des valeurs dudit paramètre biologique est supérieure à une valeur seuil maximale de référence ou inférieure à une valeur seuil minimale de référence et/ou si lesdites valeurs ne fluctuent pas autour d'une valeur moyenne de référence, fournir la valeur d'au moins un autre paramètre biologique statistiquement lié audit paramètre biologique et/ou en déduire si le sujet souffre ou est susceptible de souffrir de la maladie.

La méthode d'évaluation de l'état de dérive d'un sujet et/ou d'aide au diagnostic d'une maladie ou à l'évaluation du risque de souffrir d'une maladie chez un sujet, peut par exemple comprendre les étapes suivantes :
a) fournir la valeur d'au moins un paramètre biologique mesurée à au moins deux instants t1 et t2 étalés dans le temps dans un échantillon biologique du sujet, pour obtenir au moins deux valeurs v1 et v2,
b) déterminer si les valeurs obtenues à l'étape a) fluctuent autour d'une valeur moyenne de référence, et
c) si lesdites valeurs ne fluctuent pas autour d'une valeur moyenne de référence, fournir la valeur d'au moins un autre paramètre biologique statistiquement lié audit paramètre biologique et/ou en déduire si le sujet souffre ou est susceptible de souffrir de la maladie.

Les différents termes sont tels que définis ci-dessus.

En particulier, la fluctuation des valeurs autour d'une valeur moyenne de référence peut être évaluée comme défini ci-dessus dans la section « *Méthode d'analyse de la dérive métabolique d'au moins un paramètre biologique chez un sujet* »*.*

La valeur moyenne de référence est de préférence une valeur moyenne de référence optimisée telle que définie ci-dessus.

Dans les méthodes ci-dessus, le paramètre biologique quantitatif et/ou ledit autre paramètre biologique statistiquement lié peut être un marqueur direct ou indirectement d'une maladie ou du risque de souffrir d'une maladie.

A titre d'exemple, le paramètre biologique anti-thyroperoxydase est un marqueur de la thyroidite d'Hashimoto, et peut également être liée à la une maladie de basdow ou encore en plus faible quantité, il peut être lié à un lupus, une polyarthrite rhumatoide, une hépatite C ou encore un cancer du sein ; le paramètre biologique consistant en la présence d'anticorps *anti-candida albicans* est un marqueur d'une infection au Candida Albicans ;ou encore le paramètre biologique consistant en la présence d' anticorps anti-*helicobacter pylori* sont un marqueur d'une infection à Hélicobacter Pylori.

Dans un mode de réalisation préféré, la méthode telle que définie ci-dessus est caractérisée en ce que ledit paramètre biologique est le volume plaquettaire moyen, ledit paramètre biologique statistiquement lié étant la thyroxine libre et ladite maladie étant l'hypothyroïdie et/ou une maladie de Basedow.

Par exemple, si le volume plaquettaire moyen est en dérive basse, la méthode peut comprendre la mesure de thyroxine libre (T4 libre). La probabilité d'avoir alors une valeur de thyroxine libre hors tolérance est en effet supérieure à 30% dans la population. Si le volume plaquettaire moyen est en dérive basse et/ou si la thyroxine T4 libre est effectivement hors tolérance, en déduire que le sujet souffre ou est susceptible de souffrir d'hypothyroïdie et/ou d'une Thyroïdite d'Hashimoto et/ou d'une maladie de Basedow. La méthode peut alors comprendre la mise en œuvre d'une méthode de diagnostic, de préférence *in vitro,* de l'hypothyroïdie et/ou d'une Thyroïdite d'Hashimoto et/ou d'une maladie de Basedow.

Dans un autre mode de réalisation, la méthode telle que définie ci-dessus est caractérisée en ce que ledit paramètre biologique est un indicateur multi-paramètre défini par le nombre de globules blancs total divisé par le nombre de globules rouges et ladite maladie est une infection par *Candida albicans,* par exemple une candidose invasive.

Le nombre de globules blancs total divisé par le nombre de globules rouges est en effet un indicateur beaucoup plus significatif que le nombre de leucocytes ou encore de lymphocytes pour estimer le risque d'une réponse positive aux anticorps anti-Candida *albicans* et donc du risque de souffrir d'une maladie liée à une infection par *Candida albicans.*

Dans un autre mode de réalisation, la méthode telle que définie ci-dessus est caractérisée en ce que ledit paramètre biologique est un indicateur multi-paramètre défini par le nombre de lymphocytes divisé par le nombre de polynucléaires neutrophiles et ladite maladie est une infection par le Cytomégalovirus (CMV).

Le nombre de lymphocytes divisé par le nombre de polynucléaires neutrophiles est en effet un indicateur plus significatif pour détecter une réponse positive aux anticorps IgG anti-cytomégalovirus, que par exemple le nombre de lymphocytes seul, ou le nombre de polynucléaires neutrophiles seuls. Il est alors possible d'évaluer la dérive de cet indicateur afin d'améliorer encore la précision de détection d'une infection par le Cytomégalovirus.

### Programme d'ordinateur

La présente invention a également pour objet un programme d'ordinateur comprenant des instructions de code de programme pour l'exécution des étapes de l'une des méthodes telles que définies ci-dessus, lorsque ledit programme est exécuté sur ordinateur.

La présente invention a donc pour objet un programme d'ordinateur comprenant des instructions de code de programme pour l'exécution des étapes de la méthode d'analyse de la dérive métabolique d'au moins un paramètre biologique quantitatif chez un sujet, telle que définie ci-dessus, lorsque ledit programme est exécuté sur ordinateur, en particulier des étapes b) et c).

La présente invention a donc pour objet un programme d'ordinateur comprenant des instructions de code de programme pour l'exécution des étapes de la méthode pour définir une valeur moyenne de référence et un écart-type de référence optimisés d'un paramètre biologique X, telle que définie ci-dessus, lorsque ledit programme est exécuté sur ordinateur, en particulier des étapes b), c) et d) ou des étapes b), c), c') et d).

La présente invention a donc pour objet un programme d'ordinateur comprenant des instructions de code de programme pour l'exécution des étapes de la méthode pour optimiser une cohorte de sujets en vue de l'étude d'un paramètre biologique X, telle que définie ci-dessus, lorsque ledit programme est exécuté sur ordinateur, en particulier de l'étape b).

Il est également décrit un programme d'ordinateur comprenant des instructions de code de programme pour l'exécution d'une, plusieurs ou des étapes de la méthode d'évaluation de l'état de dérive d'un sujet et/ou d'aide au diagnostic d'une maladie ou à l'évaluation du risque de souffrir d'une maladie chez un sujet, telle que définie ci-dessus, lorsque ledit programme est exécuté sur ordinateur, en particulier des étapes b) et c).

Il est également décrit un support d'enregistrement lisible par ordinateur sur lequel est enregistrée un programme d'ordinateur comprenant des instructions de code de programme pour l'exécution d'une ou plusieurs étapes de l'une des méthodes telles que définies ci-dessus, le programme d'ordinateur étant de préférence tel que défini ci-dessus.

Dans un mode de réalisation avantageux, le programme d'ordinateur comprend des instructions de code de programme pour :
- enregistrer au fur et à mesure d'autres paramètres biologiques,
- affiner la moyenne et écart-type optimisé de chaque paramètre biologique,
- rechercher de manière dynamique au moins un autre paramètre biologique influent, et/ou
- produire en automatique une ordonnance modifiable.

D'autres caractéristiques et avantages de l'invention ressortiront mieux des exemples qui suivent, donnés à titre illustratif et non limitatif.

### Figures

Figure 1 : Analyse de la dérive métabolique des leucocytes chez un sujet. Moyenne optimisée : 5561.7/mm3, Ecart type optimisée : 1405.8/mm3, Valeur seuil de référence haute : 10500/mm3, Valeur seuil de référence basse : 4000/mm3.
Figure 2 : Analyse de la dérive métabolique du VGM chez un sujet. Moyenne optimisée : 87,1, Ecart type optimisée : 4,9 g/dl, Valeur seuil de référence haute : 97 g/dl, Valeur seuil de référence basse : 82 g/dl.
Figure 3 : Analyse de la dérive métabolique de l'hématocrite chez un sujet. Moyenne optimisée : 43,7 %, Ecart type optimisée : 1,05 %, Valeur seuil de référence haute : 47 %, Valeur seuil de référence basse : 37 %.
Figure 4 : Analyse de la dérive métabolique des polynucléaires neutrophiles chez un sujet. Moyenne optimisée : 2931,9 mm3, Ecart type optimisée : 1016,8 mm3, Valeur seuil de référence maximale : 7000 mm3, Valeur seuil de référence minimale : 1500 mm3.

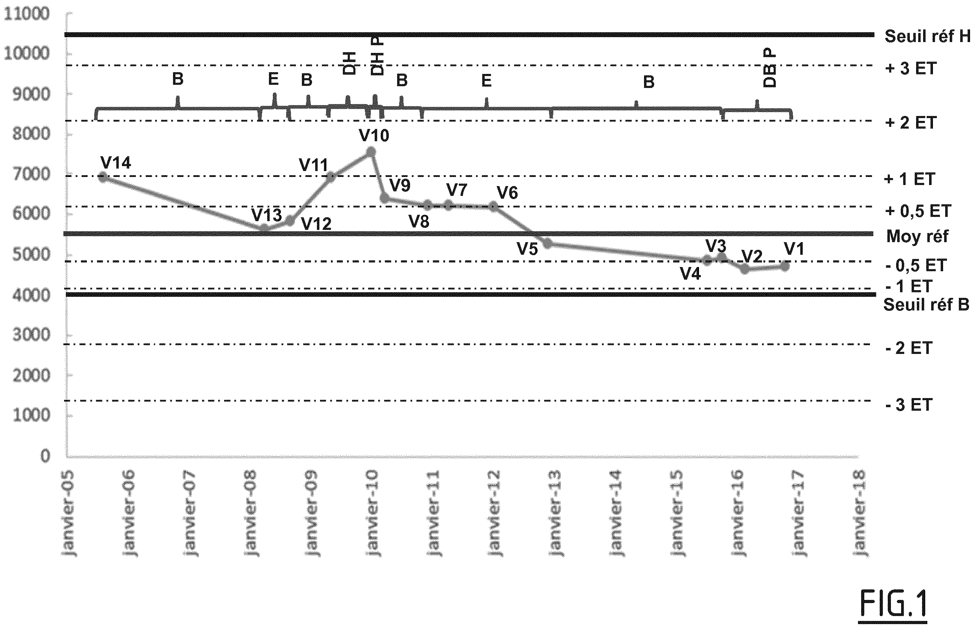

Dans les figures 1 à 4, « B » signifie un état de dérive « Bon », « E » un état de dérive « Excellent », « DH » un état de dérive haute, « DB » un état de dérive basse, « DB P » un état de dérive basse potentielle, « DH P » un état de dérive haute potentielle ; « DM imp » un état de dérive basse importante ; « ET » pour « écart-type de référence optimisé » ; « Seuil Réf H » : seuil de référence maximal définie par la HAS (Haute Autorité de Santé) ; « Seuil Réf B » : seuil de référence minimal définie par la HAS ; Moy réf : moyenne de référence optimisée.

### EXAMPLES

### Exemple 1 : Analyse de la dérive métabolique de la vitamine B12 Matériel et Méthodes

### (i) Détermination de la valeur moyenne de référence optimisée et de l'écart-type de référence optimisé pour la vitamine B12

Le groupe initial de sujets comprend 67 sujets.

Les paramètres biologiques étudiés comprennent, entre autres, la vitamine B12, la vitamine D, la numération hématies, VGM, Taux d'hémoglobine, Numération Leucocyte, polynucléaire neutrophile, polynucléaire éosinophile, polynucléaire basophile, Lymphocytes, Monocytes, Plaquette, Volume Plaquettaire moyen, Glycémie à jeun, l'hémoglobine glyquée, l'acide urique, la créatinine, les TGO et TGP, les Gamma GT, la Bilirubine, la Bilirubine libre, la Bilirubine Conjuguée, HLA DQ, HLA A, HLA B, HLA C, HLA DR.

Les valeurs des paramètres biologiques dans le groupe initial sont fournies à un instant t.

Les paramètres biologiques identifiés comme étant statistiquement liés à la concentration en vitamine B12 comprennent notamment la vitamine D, HLA-DQ3, le nombre de Hématies et le taux d'Hémoglobine.

La concentration en vitamine D est hors tolérance chez plus de 61 à 72% des patients présentant une dérive basse de la vitamine B12. 37 sujets présentant une concentration en vitamine D en dérive ou en potentielle dérive ou hors tolérance sont donc écartés du groupe pour le calcul de la moyenne de référence et de l'écart-type de référence.

La moyenne et l'écart-type sont alors calculés dans le groupe de 30 sujets restant.

### (ii) Analyse de la dérive métabolique

Les valeurs de vitamine B12 mesurées au cours de temps chez ce patient sont les suivantes : v1 = 250pg/ml ; v2 = 300pg/ml ; v3 = 278pg/ml (mesures espacées de 3 mois).

La valeur v1 est égale à 1,36 écart-type de référence optimisé de la moyenne de référence optimisée, la valeur v2 égale à 1,09 et la valeur v3 égale à 1,21.

La valeur absolue de la différence entre la moyenne de référence optimisée et la moyenne v1 + v2 est égale à 225.5 pg/ml, c'est-à-dire égale à 1.22 x l'écart-type de référence optimisé.

Les valeurs v1 et v2 sont donc en dérive.

La valeur absolue de la différence entre la moyenne de référence optimisée et la moyenne v2+v3 est égale à 211.5 pg/ml, c'est-à-dire égale à 1.15 x l'écart-type de référence optimisé.

Les valeurs v2 et v3 sont donc en dérive.

De ce fait, il est également possible de regarder la dérive de la moyenne de v1, v2 et v3.

La valeur absolue de la différence entre la moyenne de référence optimisée et la moyenne v1 + v2 + v3 est égale à 224.5 pg/ml, c'est-à-dire égale à 1.22 x l'écart-type de référence optimisé.

La vitamine B12 est donc en dérive. De ce fait, la Vitamine D est probablement en dérive et son taux est mesuré pour le vérifier.

### Résultats

### (1) Valeur moyenne de référence optimisée et de l'écart-type de référence optimisé pour la vitamine B12

La valeur moyenne et l'écart-type de référence de la vitamine B12 optimisés, en particulier calculés dans le sous-groupe ne comprenant pas de sujets présentant une concentration en vitamine D en dérive ou en potentielle dérive ou hors tolérance, sont respectivement de 500.5pg/ml et 184.5pg/ml.

### (2) Analyse de la dérive métabolique de la vitamine B12 chez un sujet

La valeur moyenne de référence optimisée et l'écart-type de référence optimisés obtenus précédemment sont utilisés pour évaluer la dérive métabolique de la vitamine B12 chez un sujet.

Les valeurs de vitamine B12 mesurées au cours de temps chez ce patient sont les suivantes : 250pg/ml ; 300pg/ml ; 278pg/ml (mesures espacées de 3 mois).

La plage de valeurs de référence pour la vitamine B12 utilisée en France est comprise de 180pg/ml à 914pg/ml.

Par ailleurs, en utilisant comme seuils de tolérance classique la valeur moyenne optimisée ± 2 écart-type optimisés, on obtient une valeur seuil minimale optimisée de 131.5 et une valeur seuil maximale optimisée de 869.5.

Dans les deux cas, on constate donc que les valeurs mesurées pour ce sujet sont comprises dans la plage de valeurs de référence selon les méthodes actuellement utilisés.

Or, les valeurs mesurées du taux de vitamine B12 ne fluctuent pas autour de la valeur moyenne de référence optimisée. Plus, précisément le taux moyen de ces trois points est à 1.2 écart type de la valeur moyenne de référence optimisée. La vitamine B12 est donc en dérive, ce qui n'est pas détecté par les méthodes actuellement utilisées.

La vitamine D étant statistiquement liée à la vitamine B12, la concentration en vitamine D chez ce sujet est mesurée au cours du temps. De la même façon, les valeurs obtenues ne fluctuent probablement pas autour de la moyenne de référence et sont probablement en dérive ou en potentielle dérive.

Le sujet reçoit alors un traitement à base de de vitamine D, par exemple à raison de 1 ampoule de 100 000UI tous les 3 mois et de la vitamine B12 par exemple en injection ou voie orale.

Sa vitamine B12 et sa concentration en vitamine D sont mesurées au cours du temps jusqu'à obtenir une correction suffisante.

### Exemple 2 : Analyse de la dérive métabolique de différents paramètres biologiques d'un même sujet

La figure 1 montre la dérive chronique des leucocytes pendant 1 an, avant un retour à un état équilibré. Les valeurs mesurées des leucocytes commence cependant à devenir faible. Ce paramètre biologique est donc à surveiller.

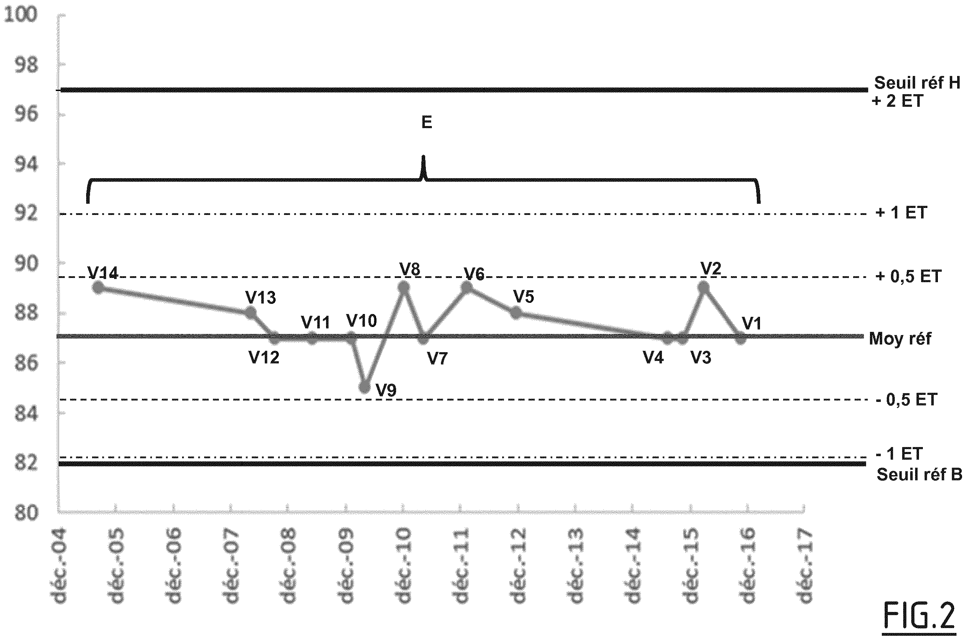

La figure 2 concernant l'analyse de la dérive métabolique de VGM. Aucune dérive de ce paramètre biologique n'est observée depuis 12 ans. Ceci montre qu'un paramètre biologique peut être très stable au cours du temps.

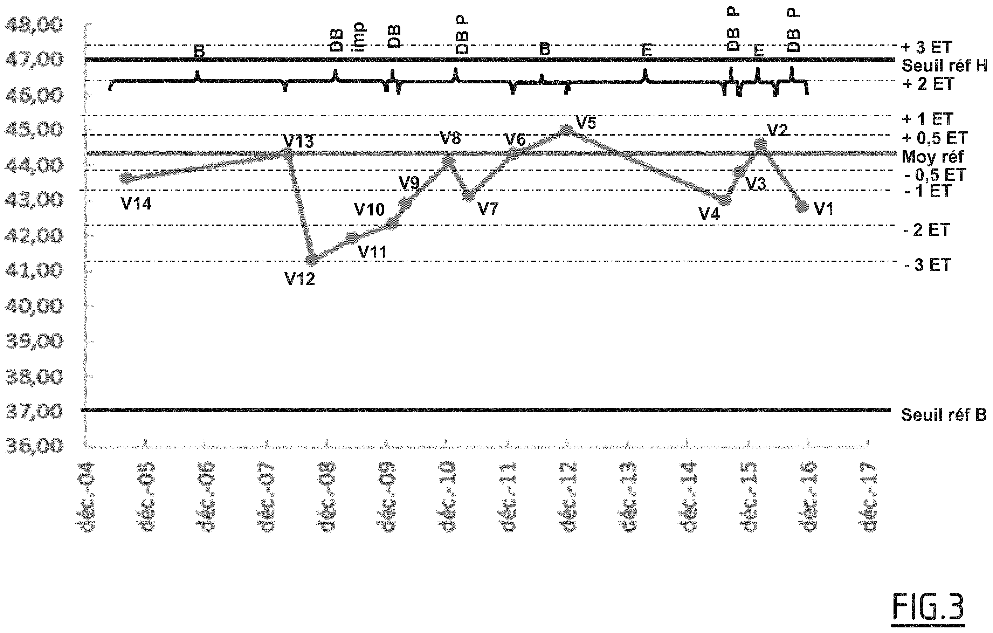

La figure 3 montre une dérive importante chronique de l'hématocrite pendant 1an ½, avant un retour à un état stable.

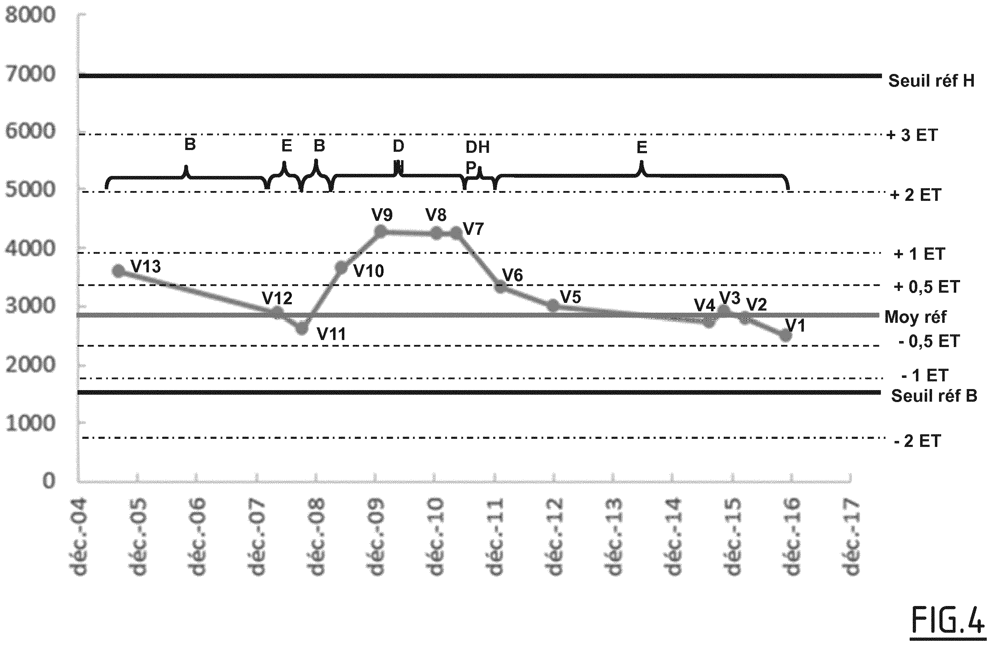

La figure 4 montre une dérive chronique de Polynucléaires Neutrophiles pendant 1an ½, avant un retour à un état excellent.

## Revendications

1. Méthode d'analyse de la dérive métabolique d'au moins un paramètre biologique quantitatif chez un sujet, **caractérisée en ce qu'**elle comprend les étapes suivantes :
a) fournir les valeurs d'au moins un paramètre biologique quantitatif mesurées à au moins deux instants t1 et t2 étalés dans le temps, pour obtenir au moins deux valeurs v1 et v2,
b) déterminer si lesdites valeurs fluctuent autour d'une valeur moyenne de référence pour ledit paramètre biologique quantitatif,
c) si lesdites valeurs ne fluctuent pas autour de ladite valeur moyenne de référence, en conclure que ledit paramètre biologique est en dérive ou en potentielle dérive, et
lorsque ledit paramètre biologique quantitatif est en potentielle dérive ou en dérive, fournir la valeur d'au moins un autre paramètre biologique statistiquement lié audit paramètre biologique en dérive ou en potentielle dérive et/ou mettre en œuvre une méthode de diagnostic d'une maladie ou du risque de souffrir d'une maladie, ladite maladie étant associée audit paramètre biologique en potentielle dérive ou en dérive ou à un paramètre biologique statistiquement lié audit paramètre biologique en dérive,
**caractérisée en ce que** lesdites valeurs fluctuent autour d'une valeur moyenne de référence pour ledit paramètre biologique quantitatif si la moyenne desdites valeurs est égale ou équivalente à ladite valeur moyenne de référence, et
**caractérisée en ce que** (i) ledit paramètre biologique est le volume plaquettaire moyen, ledit paramètre biologique statistiquement lié est la thyroxine libre et ladite maladie est l'hypothyroïdie et/ou une maladie de Basedow ou (ii) ledit paramètre biologique est un indicateur multi-paramètre défini par le nombre de globules blancs total divisé par le nombre de globules rouges et ladite maladie est une infection par *Candida albicans,* ou (iii) ledit paramètre biologique est un indicateur multi-paramètre défini par le nombre de lymphocytes divisé par le nombre de polynucléaires neutrophiles et ladite maladie est une infection par le Cytomégalovirus,
**caractérisée en ce que** la valeur moyenne de référence est définie selon la méthode de la revendication 4.

2. Méthode d'analyse selon la revendication 1, **caractérisée en ce que** :
- ledit paramètre biologique quantitatif est en dérive si :
∘ la valeur absolue de la différence entre (i) la moyenne desdites valeurs et (ii) la valeur moyenne de référence est supérieure ou égale à 1 écart-type de référence, ou
∘ la valeur absolue de la différence entre (i) la moyenne desdites valeurs et (ii) la valeur moyenne de référence est supérieure ou égale à 0,5 écart-type de référence et inférieure à 1 écart-type de référence et la valeur absolue de la différence entre (i) au moins une desdites valeurs et (ii) la valeur moyenne de référence est supérieure ou égale à 2 écart-type de référence, ou
∘ au moins une desdites valeurs est supérieure ou égale à une valeur seuil maximale de référence et/ou au moins une desdites valeurs est inférieure ou égale à une valeur seuil minimale de référence,
et/ou
- ledit paramètre biologique quantitatif est en potentielle dérive si :
∘ la valeur absolue de la différence entre (i) au moins une desdites valeurs ou la moyenne desdites valeurs et (ii) la valeur moyenne de référence est supérieure ou égale à 0,5 écart-type de référence et inférieure à 1 écart-type de référence, et
∘ si la valeur absolue de la différence entre (i) chacune desdites valeurs et (ii) la valeur moyenne de référence est inférieure à 2 écart-type de référence.

3. Méthode d'analyse selon l'une des revendications 1 à 2, **caractérisée en ce que**, lorsque ledit paramètre biologique quantitatif est en potentielle dérive ou en dérive, ladite méthode comprend une étape ultérieure de mesure de la valeur dudit paramètre biologique quantitatif à un autre instant tᵢ.

4. Méthode pour définir une valeur moyenne de référence et un écart-type de référence optimisés d'un paramètre biologique quantitatif X, comprenant :
a) fournir (i) une valeur du paramètre biologique quantitatif X mesurée à un instant donné ou au moins deux valeurs du paramètre biologique quantitatif X mesurées à différents instants séparés dans le temps et (ii) au moins deux valeurs d'au moins un autre paramètre biologique quantitatif mesurées à différents instants séparés dans le temps, pour chaque sujet d'un groupe consistant en au moins 50 sujets,
b) identifier le ou les paramètres biologiques quantitatifs statistiquement liés au paramètre biologique quantitatif X à partir d'une desdites valeurs et/ou de la moyenne desdites valeurs du paramètre biologique quantitatif X et d'une desdites valeurs ou de la moyenne desdites valeurs d'au moins un autre paramètre biologique quantitatif,
c) pour chaque paramètre biologique identifié à l'étape b), écarter du groupe les sujets chez lesquels ce paramètre biologique est en dérive ou potentielle dérive, afin de définir un sous-groupe de sujets,
c') optionnellement, fournir au moins deux valeurs d'au moins un paramètre biologique sérique mesurées à des instants étalés dans le temps pour chaque sujet du sous-groupe défini à l'étape c) et écarter du sous-groupe les sujets chez lesquels ce paramètre biologique sérique est en dérive ou potentielle dérive, afin de définir un deuxième sous-groupe de sujets, et
d) définir la moyenne et l'écart-type du paramètre biologique X dans le sous-groupe de sujets défini à l'étape c) ou dans le deuxième sous-groupe de sujets défini à l'étape c'),
**caractérisée en ce que** le paramètre biologique est en dérive ou potentielle dérive à l'étape c) si la moyenne des valeurs de ce paramètre biologique n'est pas égale ou équivalente à la valeur moyenne de référence pour ce paramètre biologique, et
**caractérisée en ce qu'**un paramètre biologique en dérive ou en potentielle dérive correspond à un état de santé anormal ou potentiellement anormal pour ce paramètre biologique,
**caractérisée en ce que** (i) ledit paramètre biologique X est le volume plaquettaire moyen et ledit paramètre biologique statistiquement lié est la thyroxine libre ou (ii) ledit paramètre biologique X est un indicateur multi-paramètre défini par le nombre de globules blancs total divisé par le nombre de globules rouges, ou (iii) ledit paramètre biologique X est un indicateur multi-paramètre défini par le nombre de lymphocytes divisé par le nombre de polynucléaires neutrophiles.

5. Méthode selon la revendication 4, **caractérisée en ce que** l'étape a) comprend en outre de fournir la valeur d'au moins un autre paramètre biologique qualitatif mesurée à un instant donné et/ou des valeurs d'au moins un autre paramètre biologique qualitatif mesurées à différents instants séparés dans le temps, **en ce que** l'étape b) comprend en outre d'identifier le ou les paramètres biologiques qualitatifs statistiquement liés au paramètre biologique quantitatif X à partir d'une desdites valeurs et/ou de la moyenne desdites valeurs du paramètre biologique quantitatif X et d'une ou desdites valeurs d'au moins un autre paramètre biologique qualitatif, et **en ce que** l'étape c) comprend en outre d'écarter du groupe les sujets chez lesquels ce paramètre biologique qualitatif est influent.

6. Méthode pour optimiser une cohorte de sujets en vue de l'étude d'un paramètre biologique X, comprenant les étapes de :
a) fournir au moins deux valeurs mesurées à différents instants séparés dans le temps et/ou une moyenne d'au moins deux valeurs mesurées à différents instants étalés dans le temps d'au moins un paramètre biologique quantitatif statistiquement lié au paramètre biologique X, pour chaque sujet d'une cohorte de sujets,
b) pour chaque paramètre biologique quantitatif, écarter de la cohorte ou placer dans un sous-groupe les sujets chez lesquels ce paramètre biologique est en dérive ou potentielle dérive, afin d'obtenir une cohorte optimisée,
c) optionnellement, fournir la valeur à un instant donné, au moins deux valeurs mesurées à différents instants étalés dans le temps et/ou une moyenne d'au moins deux valeurs mesurées à différents instants étalés dans le temps du paramètre biologique X dans la cohorte optimisée définie à l'étape b),
**caractérisée en ce que** le paramètre biologique est en dérive ou potentielle dérive à l'étape b) si la moyenne des valeurs de ce paramètre biologique n'est pas égale ou équivalente à la valeur moyenne de référence pour ce paramètre biologique, et
**caractérisée en ce qu'**un paramètre biologique en dérive ou en potentielle dérive correspond à un état de santé anormal ou potentiellement anormal pour ce paramètre biologique,
**caractérisée en ce que** (i) ledit paramètre biologique X est le volume plaquettaire moyen et ledit paramètre biologique statistiquement lié est la thyroxine libre ou (ii) ledit paramètre biologique X est un indicateur multi-paramètre défini par le nombre de globules blancs total divisé par le nombre de globules rouges, ou (iii) ledit paramètre biologique X est un indicateur multi-paramètre défini par le nombre de lymphocytes divisé par le nombre de polynucléaires neutrophiles.

7. Méthode selon la revendication 6, **caractérisée en ce que** l'étape a) comprend en outre de fournir une valeur mesurée à un instant donné ou au moins deux valeurs mesurées à des instants séparés dans le temps d'au moins un paramètre biologique qualitatif statistiquement lié au paramètre biologique X, pour chaque sujet de la cohorte de sujets, et **en ce que** l'étape b) comprend en outre, pour chaque paramètre biologique qualitatif, d'écarter de la cohorte ou placer dans un sous-groupe les sujets chez lesquels ce paramètre biologique qualitatif est influent.

8. Programme d'ordinateur comprenant des instructions de code de programme pour l'exécution des étapes d'une méthode selon les revendications 1 à 7, lorsque ledit programme est exécuté sur ordinateur.

## Patentansprüche

1. Verfahren zur Analyse der metabolischen Abweichung mindestens eines quantitativen biologischen Parameters bei einem Subjekt, **dadurch gekennzeichnet, dass** es die folgenden Schritte umfasst:
a) Bereitstellen der Werte mindestens eines quantitativen biologischen Parameters, gemessen zu mindestens zwei Zeitpunkten t1 und t2, die im Zeitverlauf verteilt sind, um mindestens zwei Werte v1 und v2 zu erhalten,
b) Bestimmen, ob die besagten Werte um einen Referenzmittelwert für den besagten quantitativen biologischen Parameter schwanken,
c) wenn die besagten Werte nicht um den besagten Referenzmittelwert schwanken, daraus schließen, dass der besagte biologische Parameter in Abweichung oder in potenzieller Abweichung ist, und wenn der besagte quantitative biologische Parameter in potenzieller Abweichung oder in Abweichung ist, Bereitstellen des Wertes mindestens eines anderen biologischen Parameters, der statistisch mit dem besagten biologischen Parameter in Abweichung oder in potenzieller Abweichung verknüpft ist, und/oder Durchführen eines Verfahrens zur Diagnose einer Krankheit oder des Risikos, an einer Krankheit zu leiden, wobei die besagte Krankheit mit dem besagten biologischen Parameter in potenzieller Abweichung oder in Abweichung oder mit einem biologischen Parameter assoziiert ist, der statistisch mit dem besagten biologischen Parameter in Abweichung verknüpft ist,
**dadurch gekennzeichnet, dass** die besagten Werte um einen Referenzmittelwert für den besagten quantitativen biologischen Parameter schwanken, wenn der Mittelwert der besagten Werte gleich oder äquivalent zu dem besagten Referenzmittelwert ist, und
**dadurch gekennzeichnet, dass** (i) der besagte biologische Parameter das mittlere Thrombozytenvolumen ist, der besagte statistisch verknüpfte biologische Parameter freies Thyroxin ist und die besagte Krankheit Hypothyreose und/oder eine Basedow-Krankheit ist oder (ii) der besagte biologische Parameter ein Multi-Parameter-Indikator ist, der durch die Anzahl der weißen Blutkörperchen insgesamt geteilt durch die Anzahl der roten Blutkörperchen definiert ist, und die besagte Krankheit eine Infektion durch *Candida albicans* ist, oder (iii) der besagte biologische Parameter ein Multi-Parameter-Indikator ist, der durch die Anzahl der Lymphozyten geteilt durch die Anzahl der neutrophilen polynukleären Zellen definiert ist, und die besagte Krankheit eine Infektion durch das Cytomegalovirus ist,
**dadurch gekennzeichnet, dass** der Referenzmittelwert gemäß dem Verfahren nach Anspruch 4 definiert wird.

2. Analyseverfahren nach Anspruch 1, **dadurch gekennzeichnet, dass**:
- der besagte quantitative biologische Parameter in Abweichung ist, wenn:
∘ der Absolutwert der Differenz zwischen (i) dem Mittelwert der besagten Werte und (ii) dem Referenzmittelwert größer oder gleich 1 Referenz-Standardabweichung ist, oder
∘ der Absolutwert der Differenz zwischen (i) dem Mittelwert der besagten Werte und (ii) dem Referenzmittelwert größer oder gleich 0,5 Referenz-Standardabweichung und kleiner als 1 Referenz-Standardabweichung ist und der Absolutwert der Differenz zwischen (i) mindestens einem der besagten Werte und (ii) dem Referenzmittelwert größer oder gleich 2 Referenz-Standardabweichungen ist, oder
∘ mindestens einer der besagten Werte größer oder gleich einem maximalen Referenz-Schwellenwert ist und/oder mindestens einer der besagten Werte kleiner oder gleich einem minimalen Referenz-Schwellenwert ist,
und/oder
- der besagte quantitative biologische Parameter in potenzieller Abweichung ist, wenn:
∘ der Absolutwert der Differenz zwischen (i) mindestens einem der besagten Werte oder dem Mittelwert der besagten Werte und (ii) dem Referenzmittelwert größer oder gleich 0,5 Referenz-Standardabweichung und kleiner als 1 Referenz-Standardabweichung ist, und
∘ wenn der Absolutwert der Differenz zwischen (i)jedem der besagten Werte und (ii) dem Referenzmittelwert kleiner als 2 Referenz-Standardabweichungen ist.

3. Analyseverfahren nach einem der Ansprüche 1 bis 2, **dadurch gekennzeichnet, dass**, wenn der besagte quantitative biologische Parameter in potenzieller Abweichung oder in Abweichung ist, das besagte Verfahren einen nachfolgenden Schritt des Messens des Wertes des besagten quantitativen biologischen Parameters zu einem anderen Zeitpunkt tᵢ umfasst.

4. Verfahren zum Definieren eines optimierten Referenzmittelwerts und einer optimierten Referenz-Standardabweichung eines quantitativen biologischen Parameters X, umfassend:
a) Bereitstellen (i) eines Wertes des quantitativen biologischen Parameters X, gemessen zu einem gegebenen Zeitpunkt, oder mindestens zweier Werte des quantitativen biologischen Parameters X, gemessen zu verschiedenen im Zeitverlauf getrennten Zeitpunkten, und (ii) mindestens zweier Werte mindestens eines anderen quantitativen biologischen Parameters, gemessen zu verschiedenen im Zeitverlauf getrennten Zeitpunkten, für jedes Subjekt einer Gruppe, bestehend aus mindestens 50 Subjekten,
b) Identifizieren des oder der quantitativen biologischen Parameter, die statistisch mit dem quantitativen biologischen Parameter X verknüpft sind, ausgehend von einem der besagten Werte und/oder von dem Mittelwert der besagten Werte des quantitativen biologischen Parameters X und von einem der besagten Werte oder von dem Mittelwert der besagten Werte mindestens eines anderen quantitativen biologischen Parameters,
c) für jeden in Schritt b) identifizierten biologischen Parameter, Ausschließen der Subjekte, bei denen dieser biologische Parameter in Abweichung oder potenzieller Abweichung ist, aus der Gruppe, um eine Untergruppe von Subjekten zu definieren,
c') optional, Bereitstellen mindestens zweier Werte mindestens eines biologischen Serumparameters, gemessen zu im Zeitverlauf verteilten Zeitpunkten, für jedes Subjekt der in Schritt c) definierten Untergruppe, und Ausschließen der Subjekte, bei denen dieser biologische Serumparameter in Abweichung oder potenzieller Abweichung ist, aus der Untergruppe, um eine zweite Untergruppe von Subjekten zu definieren, und
d) Definieren des Mittelwerts und der Standardabweichung des biologischen Parameters X in der in Schritt c) definierten Untergruppe von Subjekten oder in der in Schritt c') definierten zweiten Untergruppe von Subjekten,
**dadurch gekennzeichnet, dass** der biologische Parameter in Schritt c) in Abweichung oder potenzieller Abweichung ist, wenn der Mittelwert der Werte dieses biologischen Parameters nicht gleich oder äquivalent zu dem Referenzmittelwert für diesen biologischen Parameter ist, und
**dadurch gekennzeichnet, dass** ein biologischer Parameter in Abweichung oder in potenzieller Abweichung einem anormalen oder potenziell anormalen Gesundheitszustand für diesen biologischen Parameter entspricht,
**dadurch gekennzeichnet, dass** (i) der besagte biologische Parameter X das mittlere Thrombozytenvolumen ist und der besagte statistisch verknüpfte biologische Parameter freies Thyroxin ist oder (ii) der besagte biologische Parameter X ein Multi-Parameter-Indikator ist, der durch die Anzahl der weißen Blutkörperchen insgesamt geteilt durch die Anzahl der roten Blutkörperchen definiert ist, oder (iii) der besagte biologische Parameter X ein Multi-Parameter-Indikator ist, der durch die Anzahl der Lymphozyten geteilt durch die Anzahl der neutrophilen polynukleären Zellen definiert ist.

5. Verfahren nach Anspruch 4, **dadurch gekennzeichnet, dass** Schritt a) ferner umfasst, den Wert mindestens eines anderen qualitativen biologischen Parameters, gemessen zu einem gegebenen Zeitpunkt, und/oder Werte mindestens eines anderen qualitativen biologischen Parameters, gemessen zu verschiedenen im Zeitverlauf getrennten Zeitpunkten, bereitzustellen, dadurch, dass Schritt b) ferner umfasst, den oder die qualitativen biologischen Parameter, die statistisch mit dem quantitativen biologischen Parameter X verknüpft sind, ausgehend von einem der besagten Werte und/oder von dem Mittelwert der besagten Werte des quantitativen biologischen Parameters X und von einem oder den besagten Werten mindestens eines anderen qualitativen biologischen Parameters zu identifizieren, und dadurch, dass Schritt c) ferner umfasst, die Subjekte, bei denen dieser qualitative biologische Parameter einflussreich ist, aus der Gruppe auszuschließen.

6. Verfahren zum Optimieren einer Kohorte von Subjekten im Hinblick auf die Untersuchung eines biologischen Parameters X, umfassend die Schritte des:
a) Bereitstellens mindestens zweier Werte, die zu verschiedenen im Zeitverlauf getrennten Zeitpunkten gemessen wurden, und/oder eines Mittelwerts von mindestens zwei Werten, die zu verschiedenen im Zeitverlauf verteilten Zeitpunkten gemessen wurden, mindestens eines quantitativen biologischen Parameters, der statistisch mit dem biologischen Parameter X verknüpft ist, für jedes Subjekt einer Kohorte von Subjekten,
b) für jeden quantitativen biologischen Parameter, Ausschließen der Subjekte, bei denen dieser biologische Parameter in Abweichung oder potenzieller Abweichung ist, aus der Kohorte oder Einordnen derselben in eine Untergruppe, um eine optimierte Kohorte zu erhalten,
c) optional, den Wert zu einem gegebenen Zeitpunkt, mindestens zwei zu verschiedenen im Zeitverlauf verteilten Zeitpunkten gemessene Werte und/oder einen Mittelwert von mindestens zwei zu verschiedenen im Zeitverlauf verteilten Zeitpunkten gemessenen Werten des biologischen Parameters X in der in Schritt b) definierten optimierten Kohorte bereitzustellen,
**dadurch gekennzeichnet, dass** der biologische Parameter in Schritt b) in Abweichung oder potenzieller Abweichung ist, wenn der Mittelwert der Werte dieses biologischen Parameters nicht gleich oder äquivalent zu dem Referenzmittelwert für diesen biologischen Parameter ist, und
**dadurch gekennzeichnet, dass** ein biologischer Parameter in Abweichung oder in potenzieller Abweichung einem anormalen oder potenziell anormalen Gesundheitszustand für diesen biologischen Parameter entspricht,
**dadurch gekennzeichnet, dass** (i) der besagte biologische Parameter X das mittlere Thrombozytenvolumen ist und der besagte statistisch verbundene biologische Parameter das freie Thyroxin ist oder (ii) der besagte biologische Parameter X ein Mehrparameter-Indikator ist, der durch die Anzahl der gesamten weißen Blutkörperchen dividiert durch die Anzahl der roten Blutkörperchen definiert ist, oder (iii) der besagte biologische Parameter X ein Mehrparameter-Indikator ist, der durch die Anzahl der Lymphozyten dividiert durch die Anzahl der neutrophilen Granulozyten definiert ist.

7. Verfahren nach Anspruch 6, **dadurch gekennzeichnet, dass** der Schritt a) ferner umfasst, einen zu einem gegebenen Zeitpunkt gemessenen Wert oder mindestens zwei zu zeitlich getrennten Zeitpunkten gemessene Werte mindestens eines qualitativen biologischen Parameters bereitzustellen, der mit dem biologischen Parameter X statistisch verbunden ist, für jedes Subjekt der Kohorte von Subjekten, und dadurch, dass der Schritt b) ferner umfasst, für jeden qualitativen biologischen Parameter die Subjekte, bei denen dieser qualitative biologische Parameter einflussreich ist, aus der Kohorte auszuschließen oder in eine Untergruppe zu platzieren.

8. Computerprogramm, umfassend Programmcodeanweisungen für die Ausführung der Schritte eines Verfahrens nach den Ansprüchen 1 bis 7, wenn das besagte Programm auf einem Computer ausgeführt wird.

## Claims

1. Method for analysing the metabolic drift of at least one quantitative biological parameter in a subject, **characterized in that** it comprises the following steps:
a) providing the values of at least one quantitative biological parameter measured at least at two different spaced-apart times t1 and t2, to obtain at least two values v1 and v2;
b) determining whether said values fluctuate around a mean reference value for said quantitative biological parameter;
c) if said values do not fluctuate around said mean reference value, concluding therefrom that said biological parameter shows drift or potential drift, and when said quantitative biological parameter shows drift or potential drift, providing the value of at least one other biological parameter statistically related to the biological parameter showing drift or potential drift; and/or implementing a method to diagnose a disease or risk of suffering from a disease, said disease being associated with said biological parameter showing drift or potential drift or with a biological parameter statistically related to said biological parameter showing drift,
**characterized in that** said values fluctuate around a mean reference value for said quantitative biological parameter if the mean of said values is equal or equivalent to said mean reference value, and
**characterized in that** (i) said biological parameter is the mean platelet volume, said statistically related biological parameter is free thyroxine and said disease is hypothyroidism and/or a Graves-Basedow disease or (ii) said biological parameter is a multi-parameter indicator defined by the number of white blood cells divided by the number red blood cells and said disease is infection with *Candida albicans,* or (iii) said biological parameter is a multi-parameter indicator defined by the number of lymphocytes divided by the number of polymorphonuclear neutrophils and said disease is infection with Cytomegalovirus,
**characterized in that** the mean reference value is defined according to the method of claim 4.

2. The analysis method according to claim 1, **characterized in that**:
- said quantitative biological parameter shows drift if:
o the absolute value of the difference between (i) the mean of said values and (ii) the mean reference value is higher than or equal to 1 reference standard deviation; or
o the absolute value of the difference between (i) the mean of said values and (ii) the mean reference value is higher than or equal to 0.5 reference standard deviation and lower than 1 reference standard deviation, and the absolute value of the difference between (i) at least one of said values and (ii) the mean reference value is higher than or equal to 2 reference standard deviations; or
o at least one of said values is higher than or equal to a maximum reference threshold value and/or at least one of said values is lower than or equal to a minimum reference threshold value; and/or
- said quantitative biological parameter shows potential drift if:
o the absolute value of the difference between (i) at least one of said values or the mean of said values and (ii) the mean reference value is higher than or equal to 0.5 reference standard deviation and lower than 1 reference standard deviation; and
o if the absolute value of the difference between (i) each of said values and (ii) the mean reference value is lower than 2 reference standard deviations.

3. The analysis method according to one of claims 1 to 2, **characterized in that** when said quantitative biological parameter shows potential drift or drift, said method comprises a subsequent step to measure the value of said quantitative biological parameter at another time tᵢ.

4. Method for defining an optimised mean reference value and optimised reference standard deviation of a quantitative biological parameter X, comprising:
a) providing (i) a value of the quantitative biological parameter X measured at a given time or at least two values of the quantitative biological parameter X measured at different spaced-apart times and (ii) at least two values of at least one other quantitative biological parameter measured at different spaced-apart times, for each subject in a group of at least 50 subjects;
b) identifying the quantitative biological parameter(s) statistically related to the quantitative biological parameter X from one of said values and/or from the mean of said values of the quantitative biological parameter X, and from one of said values or the mean of said values of at least one other quantitative biological parameter;
c) for each biological parameter identified at step b), removing from the group those subjects in whom this biological parameter shows drift or potential drift, to define a sub-group of subjects;
c') optionally, providing at least two values of at least one serum biological parameter measured at different spaced-apart times for each subject in the sub-group defined at step c) and removing from the sub-group those subjects in whom this serum biological parameter shows drift or potential drift, to define a second sub-group of subjects; and
d) defining the mean and standard deviation of the biological parameter X in the sub-group of subjects defined at step c) or in the second sub-group of subjects defined at step c'),
**characterized in that** the biological parameter shows drift or potential drift at step c), if the mean of the values for this biological parameter is not equal or equivalent to said mean reference value for this biological parameter, and **characterized in that** a biological parameter showing drift or potential drift corresponds to an abnormal or potentially abnormal clinical condition for this biological parameter,
**characterized in that** (i) said biological parameter X is the mean platelet volume and said statistically related biological parameter is free thyroxine or (ii) said biological parameter X is a multi-parameter indicator defined by the number of white blood cells divided by the number red blood cells, or (iii) said biological parameter X is a multi-parameter indicator defined by the number of lymphocytes divided by the number of polymorphonuclear neutrophils.

5. The method according to claim 4, **characterized in that** step a) further comprises providing the value of at least one other qualitative biological parameter measured at a given time and/or values of at least one other qualitative biological parameter measured at different spaced-apart times, **in that** step b) further comprises the identification of qualitative biological parameter(s) statistically related to the quantitative biological parameter X from one of said values and/or from the mean of said values of the quantitative biological parameter X and from one or said value(s) of at least one other qualitative biological parameter, and **in that** step c) further comprises the removal from the group of those subjects in whom this qualitative biological parameter is influential.

6. Method for optimising a cohort of subjects for the purpose of studying a biological parameter X, comprising the steps of:
a) providing at least two values measured at different spaced-apart times and/or a mean of at least two values measured at different spaced-apart times of at least one quantitative biological parameter statistically related to biological parameter X, for each subject in a cohort of subjects;
b) for each quantitative biological parameter, removing from the cohort or placing in a sub-group those subjects in whom this biological parameter shows drift or potential drift, to obtain an optimised cohort;
c) optionally, providing the value at a given time, at least two values measured at different spaced-apart times and/or a mean of at least two values measured at different spaced-apart times of the biological parameter X in the optimised cohort defined at step b),
**characterized in that** the biological parameter shows drift or potential drift at step b), if the mean of the values for this biological parameter is not equal or equivalent to said mean reference value for this biological parameter, and **characterized in that** a biological parameter showing drift or potential drift corresponds to an abnormal or potentially abnormal clinical condition for this biological parameter,
**characterized in that** (i) said biological parameter X is the mean platelet volume and said statistically related biological parameter is free thyroxine or (ii) said biological parameter X is a multi-parameter indicator defined by the number of white blood cells divided by the number red blood cells, or (iii) said biological parameter X is a multi-parameter indicator defined by the number of lymphocytes divided by the number of polymorphonuclear neutrophils.

7. The method according to claim 6, **characterized in that** step a) further comprises the providing of a value measured at a given time or of at least two values measured at different spaced-apart times of at least one qualitative biological parameter statistically related to biological parameter X, for each subject in the cohort of subjects, and **in that** step b) for each qualitative biological parameter further comprises removing from the cohort or placing in a sub-group those subjects in whom this qualitative biological parameter is influential.

8. Computer programme comprising programme code instructions to execute the steps of a method according to claims 1 to 7, when said programme is executed on a computer.
